# EUROPEAN PATENT APPLICATION

(11) **EP 4 206 226 A1**
(43) Date of publication of application: **05.07.2023**
(21) Application number: 21862161.3
(22) Date of filing: 31.08.2021
(51) Int. Cl.: C07K 16/22, A61K 9/00, A61P 27/02, A61K 39/00

(54) **ANTI-VEGF HEXAMERIC ANTIBODY AND COMPOSITION COMPRISING SAME**

(30) Priority: 31.08.2020 KR 20200110439; 10.06.2021 KR 20210075180
(71) Applicant: Medytox Inc., Chungcheongbuk-do 28126 (KR)
(72) Inventor: JUN, Seungchul, Daejeon 34195 (KR); KIM, Keunsoo, Suwon-si Gyeonggi-do 16213 (KR); AN, Dong Hyun, Suwon-si Gyeonggi-do 16543 (KR); BASNET, Devi Bahadur, Suwon-si Gyeonggi-do 16445 (KR); HAN, Saeng Myung, Suwon-si Gyeonggi-do 16493 (KR)
(74) Representative: Brevalex
(86) International application number: PCT/KR2021/011686
(87) International publication number: WO 2022/045865

(57) **Abstract**

Provided are an anti-VEGF hexameric antibody, and a composition including the hexameric antibody.

## Description

### Technical Field

The present disclosure relates to a multimer of an antibody, and more particularly, to an anti-VEGF hexameric antibody and a composition including the hexameric antibody.

### Background Art

Vascular endothelial growth factor (VEGF) is a signal protein produced by cells that promote blood vessel formation, and is involved in vasculogenesis and angiogenesis. The normal function of VEGF is to create new blood vessels during embryonic development or to create new blood vessels to bypass post-traumatic or blocked blood vessels, and VEGF also contributes to disease. Cancer cells express VEGF to cause growth and metastasis, and overexpression of VEGF can cause vascular disease in the retina of the eye. VEGF is secreted from retinal epithelial cells under hypoxia and increases the differentiation of vascular endothelial cells and the permeability of retinal blood vessels, thereby contributing to the generation of new blood vessels accompanying age-related macular degeneration (AMD) or the permeability of abnormal blood vessels. Therefore, it is important to block vascular endothelial growth factor in age-related macular degeneration, and several vascular endothelial growth factor antagonists are used. For example, Bevacizumab (Avastin^{®}), Ranibizumab (Lucentis^{®}), and Aflibercept (Eylea^{®}), and the like may be used.

For the treatment of ocular diseases such as age-related macular degeneration, vascular endothelial growth factor antagonists and the like as described above are administered by intravitreal injection. In this case, however, it is desperately needed to increase the dosing intervals. To this end, attempts have been made to increase the half-life in the vitreous body by increasing the molecular weight of macromolecules. For example, International Publication No. WO 2017/117464 discloses that an anti-VEGF-A antibody and a polymer containing phosphorylcholine are included, wherein the polymer is covalently linked to the antibody at a cysteine positioned outside the variable region of the antibody, and the cysteine is an antibody conjugate added through recombinant DNA technology and may have a half-life that is more than 1.5 times higher than that of an unconjugated antibody to reduce the frequency of administration. This document discloses that antibody-biopolymer conjugates can have a molecular weight of 300 kDa to 1,750 kDa and may be administered no more often than once a month.

Meanwhile, U.S. Patent No. 9,540,442 discloses an antibody comprising an IgG hinge, CH2 and CH3 regions from the N-terminus to the C-terminus, wherein amino acids at position 279, 285 or 287 according to EU numbering in the CH2 region are mutated into cysteine residues, and the CH3 site is linked to the µ tailpiece at the C-terminus so that units of the antibody can multimerize via disulfide bonding between cysteines at the mutated position in different units and between different µ tailpieces. This document exemplifies antibodies that specifically bind to the death receptor family, such as DR4 or DR5, but does not teach or suggest antibodies that specifically bind to VEGF.

### Detailed Description of the Invention

### Technical Problem

An objective of the present disclosure is to provide an anti-VEGF hexameric antibody.

An objective of the present disclosure is to provide a pharmaceutical composition for preventing or treating angiogenesis disease including the hexameric antibody as an active ingredient.

An objective of the present disclosure is to provide an antibody composition containing the hexameric antibody at a high ratio.

### Technical solution

One aspect of the present disclosure provides a hexameric antibody in which six identical antibody monomers are linked by a covalent bond, and the antibody monomers bind specifically to a vascular endothelial growth factor (VEGF).

Another aspect of the present disclosure provides a pharmaceutical composition for preventing or treating angiogenic disease, including the hexameric antibody as an active ingredient.

Another aspect of the present disclosure provides an antibody composition containing 90% by weight or more of the hexameric antibody based on the total weight of the antibody composition.

### Advantageous Effects of Disclosure

Anti-VEGF hexameric antibodies according to the present disclosure can migrate to the retina and the choroid even with an increased molecular weight thereof, has a high residual amount in the vitreous body and retina/choroid, and an increased intraocular half-life, so that when administered intravitreally, the frequency of administration can be reduced, and can be efficiently produced with high purity due to a high hexamer ratio during antibody expression and purification.

### Brief Description of Drawings

FIG. 1 is a diagram in which the amino acid sequence of rabbit is compared with the amino acid sequence of a humanized anti-VEGF antibody.
FIG. 2 is a diagram in which the amino acid sequence of Ranibizumab is compared with the amino acid sequence of variant Ranibizumab.
FIG. 3 is a diagram showing the results of SDS-PAGE analysis after purification of the anti-VEGF hexameric antibody.
FIGS. 4A and 4B are graphs showing the VEGF binding ability of anti-VEGF monomers and hexameric antibodies.
FIG.5 is a graph showing the calcium suppression effect induced by VEGF in HUVEC cells of an anti-VEGF hexameric antibody.
FIGS. 6A, 6B, 6C, 6D, 6E, 6F, 6G, and 6H are diagrams showing the results of analyzing the aggregate content of anti-VEGF hexameric antibodies.
FIGS. 7A, 7B, and 7C are diagrams showing the binding ability of anti-VEGF hexameric antibodies to FcγRI, FcγRII, and FcγRIII.
FIG. 8 is a diagram showing the binding ability of an anti-VEGF hexameric antibody to FcRn.
FIGS. 9A, 9B, and 9C are diagrams showing human pharmacokinetic prediction results of anti-VEGF hexameric antibodies.
FIGS. 10A, 10B, and 10C are diagrams showing the rat choroidal neovascularization inhibitory effect of anti-VEGF hexameric antibodies.
FIG. 11 is a diagram showing the results of monkey retinal penetration test of anti-VEGF hexameric antibodies, wherein ILM represents an inner limiting membrane, INL represents an inner nuclear layer, ONL represents an outer nuclear layer, and Ch represents choroid.
FIGS. 12A and 12B are views showing dynamic light scattering analysis of the formation of anti-VEGF hexameric antibodies.

### Best Mode for the Invention

The term 'antibody' used herein includes a whole antibody, and any antigen-binding fragments or single chains thereof. An 'antibody' includes a glycoprotein including at least two heavy chains (H) and two light chains (L) linked by disulfide bonds, or an antigen-binding portion thereof. Each heavy chain includes a variable region of the heavy chain (VH or HV) and a constant region of the heavy chain. The constant region of the heavy chain consists of three regions: CH1, CH2 and CH3. Each light chain consists of a variable region of the light chain (VL or LV) and a constant region of the light chain. The constant region of the light chain consists of one region, that is, CL. The VH and VL regions may be subdivided into regions that are more conserved, called framework regions (FR), and hypervariable regions, called complementarity determining regions (CDRs). Each of VH and VL consists of three CDRs and four FRs arranged from the amino terminus to the carboxy terminus in the sequence of FR1, CDR1, FR2, CDR2, FR3, CDR3, FR4 (in the case of VH, HFR1, HCDR1, HFR2, HCDR2, HFR3, HCDR3, and HFR4; in the case of VL, LFR1, LCDR1, LFR2, LCDR2, LFR3, LCDR3, and LFR4. The variable regions of the heavy and light chains contain binding regions that interact with an antigen. The constant region may mediate the binding of immunoglobulins to host tissues or factors, including various cells of the immune system (for example, effector cells) and the first component (C1q) of the classical complement system.

The term "humanized antibody" used herein refers to a molecule of which an antigen-binding portion is substantially derived from an immunoglobulin from a non-human species and the remainder of the immunoglobulin structure is based on the structure and/or sequence of a human immunoglobulin. The antigen-binding portion may include the complete variable region fused onto the constant region or may include only a complementarity determining region (CDR) grafted to an appropriate framework region within the variable region. The antigen-binding portion may be wild-type or may be modified by one or more amino acid substitutions. For example, the antigen-binding portion may be modified so as to resemble human immunoglobulins more closely. Some types of humanized antibodies preserve all CDR sequences (for example, a humanized rabbit antibody that contains all 6 CDRs from a rabbit antibody). In the case of other types thereof, one or more CDRs may differ from the original antibody.

The terms 'Fc', 'Fc site', and 'Fc region' refer to a portion of the antibody molecule consisting of a hinge portion or a portion thereof, and CH2 region, and CH3 region. The Fc region of the IgG class is, for example, from the 226th cysteine to the C terminus, or from the 230th proline to the C terminus, according to EU numbering (also called EU INDEX), but is not limited thereto. The Fc site may be appropriately obtained by partially digesting IgG1, IgG2, IgG3, or IgG4 monoclonal antibody with a proteolytic enzyme such as pepsin or papain, and then re-eluting the fraction adsorbed to Protein A column or Protein G column.

The inventors of the present application found as follows: when an antibody is administered into the vitreous body, the antibody is discharged from the eyeball to the body through the anterior chamber or the posterior chamber of the eyeball, that is, the retina, due to the diffusion rate. Accordingly, they increased the molecular weight of the antibody in order to slow the rate of intraocular excretion through diffusion. By doing so, the rate of diffusion is reduced and thus the antibody is able to remain in the vitreous body and the retina for a long time. In response, they prepared the hexameric anti-VEGF antibody with high purity and high yield, and found that the hexameric antibody migrates to the retina and choroid, and the amount of the antibody remaining in the vitreous body and retina/choroid is much higher than that in the case of the monomer, and the intraocular half-life is greatly increased compared to the case of the monomer.

One aspect of the present disclosure provides a hexameric antibody in which six identical antibody monomers are linked by a covalent bond, and the antibody monomers bind specifically to a vascular endothelial growth factor (VEGF).

In embodiments, the antibody monomer may have an isotype of immunoglobulin G (IgG).

In embodiments, the antibody monomers may be linked by disulfide bonds in the CH2 region, the CH3 region, or the CH2 region and the CH3 region, in the Fc region.

In an embodiment, in the antibody monomer, amino acid number 309 may be mutated to cysteine according to Kabat numbering, a µ tailpiece may be linked to the C-terminus of Fc, or amino acid number 309 may be mutated to cysteine and µ tailpiece may be linked to C-terminus of Fc.

In an embodiment, in the antibody monomer, amino acid 310 may be mutated to leucine, amino acid 430 may be mutated to histidine, or amino acid 310 may be mutated to leucine and amino acid 430 may be mutated to histidine, according to Kabat numbering.

The position of leucine 309 in the constant region of IgG is homologous to that of cysteine 414 in IgM, and is an important position in forming a disulfide bridge between monomers in human IgM, and forming a hexamer in the absence of a J chain. In addition, the histidine 310 position, which is the adjacent amino acid, may also be mutated to leucine, which is because histidine with a positive charge can interfere with disulfide bridge bonding. A tail fragment consisting of 18 amino acids from human IgM, that is, µ tailpiece (SEQ ID NO: 61) may be added after C-terminus lysine 447 of IgG. IgM tail fragment peptide extensions may play a role in driving IgM assembly. The hydrophobic amino acids located in the front half of the tail fragment contain the essential information needed to generate the correct topology, and the assembly between monomers may be facilitated by the formation of a disulfide bond between two tail fragments.

In an embodiment, the antibody monomer may include, compared to human germ-line immunoglobulins, a variable region of a heavy chain having a heavy chain framework having one or more of Kabat numbering 13Q, 37I, 74S, 77T and 105Q; a variable region of a light chain having a light chain framework having one or more of Kabat numbering 22T, 43A, 48L or 48I, 83F and 100Q; or a combination thereof.

Additionally, the heavy chain framework may have one or more of 49A and 73D.

For example, the antibody monomer may include: a variable region of a heavy chain having one or more heavy chain frameworks of HFR1 of SEQ ID NO: 37 or SEQ ID NO: 51, HFR2 of SEQ ID NO: 38 or SEQ ID NO: 52, HFR3 of SEQ ID NO: 39 or SEQ ID NO: 53, and HFR4 of SEQ ID NO: 40; a variable region of a light chain having one or more light chain frameworks of LFR1 of SEQ ID NO: 41, LFR2 of SEQ ID NO: 42 or SEQ ID NO: 54, LFR3 of SEQ ID NO: 43, and LFR4 of SEQ ID NO: 44; or a combination thereof.

For example, the antibody monomer may include a variable region of a heavy chain having a heavy chain framework consisting of HFR1 of SEQ ID NO: 37, HFR2 of SEQ ID NO: 38, HFR3 of SEQ ID NO: 39, and HFR4 of SEQ ID NO: 40; and a variable region of a light chain having a light chain framework consisting of LFR1 of SEQ ID NO: 41, LFR2 of SEQ ID NO: 42, LFR3 of SEQ ID NO: 43, and LFR4 of SEQ ID NO: 44.

For example, the antibody monomer may include a variable region of a heavy chain having a heavy chain framework consisting of HFR1 of SEQ ID NO: 51, HFR2 of SEQ ID NO: 52, HFR3 of SEQ ID NO: 53, and HFR4 of SEQ ID NO: 40; and a variable region of a light chain having a light chain frame work consisting of LFR1 of SEQ ID NO: 41, LFR2 of SEQ ID NO: 54, LFR3 of SEQ ID NO: 43, and LFR4 of SEQ ID NO: 44.

For example, the antibody monomer may include: a variable region of a heavy chain having one or more heavy chain CDR of HCDR1 of SEQ ID NO: 45 or SEQ ID NO: 55, HCDR2 of SEQ ID NO: 46 or SEQ ID NO: 56, and HCDR3 of SEQ ID NO: 47 or SEQ ID NO: 57; a variable region of a light chain having one or more light chain CDR of LCDR1 of SEQ ID NO: 48 or SEQ ID NO: 58, LCDR2 of SEQ ID NO: 49 or SEQ ID NO: 59, and LCDR3 of SEQ ID NO: 50 or SEQ ID NO: 60; or a combination thereof.

For example, an antibody monomer may include the variable region of a heavy chain of SEQ ID NO: 30; and a variable region of a light chain of SEQ ID NO: 31.

For example, an antibody monomer may include the variable region of a heavy chain of SEQ ID NO: 32; and a variable region of a light chain of SEQ ID NO: 33.

For example, an antibody monomer may include: the variable region of a heavy chain of SEQ ID NO: 34, SEQ ID NO: 35 or SEQ ID NO: 36; and a variable region of a light chain of SEQ ID NO: 33.

In embodiments, an antibody may have a reduced or eliminated effector function. For example, the antibody may have a reduced or eliminated binding ability to one or more of FcγRI, FcγR II, FcγR III, and FcRn. For example, the antibody may have one or more mutations from among L234A, L235A, P331G, I253A, and H310A.

In the case of antibody medicaments which targets only antigen neutralization, effector functions such as ADCC of the Fc region are not required, and in the case of antibodies acting on specific parts such as the eye, it may not be desirable to last in the corresponding system for a long time. Accordingly, binding of Fcγ receptors or FcRn in the Fc region may be unnecessary. A therapeutic antibody having a reduced immune effector function which is achieved by introducing an Fc mutation of IgG1, can be developed. The so-called 'LALA' double mutations (L234A and L235A), which were first introduced by Winter's group, reduce affinity with FcγR, and as a result, an antibody dependent cellular cytotoxicity (ADCC) activity may be reduced. The complement dependent cytotoxicity (CDC) activity may also be reduced by Fc mutation. The D270A, K322A, P329A, and P331A mutations of IgG1 may reduce the binding affinity to C1q to inhibit complement activation. The P331S mutation has weak binding to C1q, and the P331G mutation has no binding to C1q, and thus CDC activity may not appear.

For example, the antibody monomer may have an Fc site of SEQ ID NO: 74 or SEQ ID NO: 75.

For example, the antibody monomer may include a heavy chain of SEQ ID NO: 69 or SEQ ID NO: 71.

For example, the antibody monomer may include a heavy chain of SEQ ID NO: 69 and a light chain of SEQ ID NO: 70.

For example, the antibody monomer may include a heavy chain of SEQ ID NO: 71 and a light chain of SEQ ID NO: 72.

In embodiments, the hexameric antibody may have a binding affinity for VEGF of K_{D}100 pM or less. For example, the antibody may have, with respect to VEGF, a binding affinity of K_{D} 50 pM or less, K_{D} 30 pM or less, K_{D} 20 pM or less, K_{D} 15 pM or less, K_{D} 10 pM or less, K_{D} 5 pM or less, K_{D} 1 pM to K_{D} 50 pM, K_{D} 1 pM to K_{D} 30 pM, K_{D} 1 pM to K_{D} 20 pM, K_{D} 1 pM to K_{D} 15 pM, K_{D} 1 pM to K_{D} 10 pM, or K_{D} 1 pM to K_{D} 5 pM.

In embodiments, the hexameric antibody may have a thermal stability of Tm 70 °C or higher. For example, the antibody may have a thermal stability of Tm 70 °C, Tm 71 °C, Tm 72 °C, Tm 73 °C, Tm 74 °C, Tm 75 °C, Tm 70 °C to 75 °C, or Tm 73 °C to 75 °C.

The hexameric antibody according to the present disclosure has the following features: although a molecular weight thereof is high, for example, about 900 kDa, the migration to the retina and the choroid may occur well; the amount of antibody remaining in the vitreous body and retina/choroid is significantly higher than that of monomers (for example, more than 3-fold in the vitreous body and more than 9-fold in the retina/choroid); and the half-life in the eye is greatly increased, for example, 2 times or more, compared to the case of a monomer.

Another aspect of the present disclosure provides a pharmaceutical composition for preventing or treating angiogenic disease, including the hexameric antibody as an active ingredient.

In an embodiment, the pharmaceutical composition may be an aqueous pharmaceutical composition.

As used herein, the term "aqueous pharmaceutical composition" refers to a composition suitable for pharmaceutical use containing an aqueous carrier. Compositions suitable for pharmaceutical use may be sterile, homogeneous and/or isotonic. The aqueous pharmaceutical composition refers to, for example, a liquid preparation or a lyophilized preparation to be reconstituted.

In embodiments, the aqueous pharmaceutical composition may include a buffer and a tonicity agent in addition to the active ingredient.

A buffer may be, for example, one that maintains the pH to be in the range of 5.0 to 7.5. For example, the pH of the aqueous pharmaceutical composition may be 5.0 to 7.5, 5.0 to 7.0, 5.0 to 6.5, 5.0 to 6.0, or 5.0 to 5.5. Examples of buffering agents include one or more of the usual ones known in the art, and are organic acid salts such as salts of citric acid, ascorbic acid, gluconic acid, carbonic acid, tartaric acid, succinic acid, acetic acid, or phthalic acid; tris, tromethamine hydrochloride or phosphate buffers; and amino acid buffers such as histidine. In an embodiment, the buffer may be a histidine buffer. For example, the buffer may have a concentration ranging from 1 mM to 50 mM, 5 mM to 50 mM, 5 mM to 40 mM, 5 mM to 30 mM, 5 mM to 20 mM, or 5 mM to 15 mM.

For example, the tonicity agent may be a salt. The salt may include one or more selected from the group consisting of NaCl, KCI, NaF, KBr, NaBr, Na₂ SO₄, NaSCN, and K₂SO₄, and may be NaCl. For example, the salt may have a concentration in the range of 50 mM to 200 mM, 75 mM to 175 mM, 100mM to 150 mM. In an embodiment, the tonicity agent may not be a sugar or sugar alcohol, or polyol. Examples of sugars or sugar alcohols or polyols are trehalose, sucrose, mannitol, sorbitol, xylitol, glucose, glycerol, and the like.

In embodiments, the aqueous pharmaceutical composition may not include at least one of a stabilizer and a surfactant. The surfactant may be a nonionic surfactant, and may be, for example, at least one selected from: polysorbates (for example, polysorbate 20 (polyoxyethylene (20) sorbitan monolaurate), polysorbate 40 (polyoxyethylene ( 20) sorbitan monopalmitate), polysorbate 60 (polyoxyethylene (20) sorbitan monostearate), polysorbate 80 (polyoxyethylene (20) sorbitan monooleate), wherein the number (20) following "polyoxyethylene" indicates the total number of oxyethylene groups (-(CH2CH2O)-)); poloxamer (PEO-PPO-PEO copolymer where PEO represents polyethylene oxide), and PPO represents polypropylene oxide), polyethylene-polypropylene glycol, polyoxyethylene compounds (for example, polyoxyethylene-stearate, polyoxyethylene alkyl ethers (alkyl: C1-C30), polyoxyethylene monolauryl ethers, alkylphenyl polyoxyethylene copolymers (alkyl: C1-C30), etc.), and sodium dodecyl sulphate (SDS). For example, the surfactant may be polysorbates (for example, polysorbate 20). The stabilizer may include one or more amino acids or salts thereof, for example, amino acids other than histidine or salts thereof. Examples of the amino acid are valine, serine, threonine, alanine, glycine, isoleucine, asparagine, glutamine, aspartic acid, and proline. In an embodiment, the aqueous pharmaceutical composition may not contain proline. In an embodiment, an aqueous pharmaceutical composition may include proline.

In embodiments, the aqueous pharmaceutical composition may comprise, may consist essentially of, or may consist of 5 mM to 50 mM histidine buffer and 50 mM to 200 mM NaCl together with an anti-VEGF antibody.

In embodiments, the aqueous pharmaceutical composition may have a viscosity of 20 cP or less at 25 °C. The viscosity may be, at 25 °C, within the range of 1 cP to 20 cP, 2 cP to 18 cP, 2 cP to 15 cP, 3 cP to 15 cP, 2 cP to 10 cP, 3 cP to 10 cP, 5 cP to 15 cP, or 5 cP to 10 cP.

In an embodiment, the pharmaceutical composition is useful for treating angiogenic ocular disease. The "angiogenic ocular disease" that can be treated using the aqueous pharmaceutical composition according to the present disclosure includes conditions, diseases, or disorders associated with ocular angiogenesis including, but not limited to, abnormal angiogenesis, choroid angiogenesis (CNV), retinal vascular permeability, retinal edema, diabetic retinopathy (especially proliferative diabetic retinopathy), diabetes macular edema, neovascular (exudative) age-related macular degeneration (AMD) including CNV associated with nAMD (neovascular AMD), sequelae associated with retinal ischemia, central retinal vein occlusion (CRVO) and posterior segment neovascularization.

In embodiments, the aqueous pharmaceutical composition may include an additional active ingredient in addition to the anti-VEGF hexameric antibody. Additional pharmacological agents may include, for example, other antibodies useful for treating ocular disease.

In embodiments, the aqueous pharmaceutical composition may be administered orally or parenterally. In the case of parenteral administration (for example, injection), intraocular administration (for example, intravitreal administration), intravenous administration, subcutaneous administration, intramuscular administration, intraperitoneal administration, endothelial administration, topical administration, intranasal administration, intrapulmonary administration, intrarectal administration, or intratumoral administration may be used. For example, intravitreal administration may be used. In an embodiment, the aqueous pharmaceutical composition may be an ophthalmic composition containing an anti-VEGF hexameric antibody, and in this case, an injection administered into the vitreous body of the eye, may be used.

In embodiments, the pharmaceutical composition according to the present disclosure may be administered at an appropriate administration cycle and/or dose in consideration of the patient's condition, including age, health condition, severity of disease, and the like, according to the judgment of an expert such as an ophthalmologist. Since the pharmaceutical composition may contain the anti-VEGF hexameric antibody at a high concentration, the amount of the active ingredient administered at one time may be increased, and thus the pharmaceutical composition may be administered less frequently, that is, in a longer cycle. For example, the pharmaceutical composition may be administered at a dosing interval of once every 3 months or more, once every 4 months, once every 5 months, or once every 6 months or more. Regarding the pharmaceutical composition, the amount of the active ingredient administered per volume may be increased. For example, the pharmaceutical composition may be administered in a dose ranging from 2 mg to 20 mg. For example, a dose of about 2 mg, 5 mg, 10 mg, 15 mg, or 20 mg may be administered. The dose may be administered in various volumes suitable for intraocular administration, such as 100 µl or less, 50 to 100 µl, or 50 µl or less.

In embodiments, the aqueous pharmaceutical composition may be in the form of a solution or lyophilized powder. The composition may be contained in a vial, an ampoule, or a pre-filled syringe. In an embodiment, lyophilization is contemplated to provide the aqueous pharmaceutical composition according to the present disclosure. Lyophilization techniques are known in the art. The lyophilizate should be reconstituted in an aqueous reconstituted solution prior to administration to patients. This step allows the antibody and other components within the lyophilizate to be re-dissolved to provide a solution suitable for injection into the patient. The volume of aqueous medium used for reconstitution determines the concentration of antibody in the resulting pharmaceutical composition. Reconstitution using a reconstituent that has a smaller volume than the volume before lyophilization, provides a more concentrated composition than before lyophilization. The reconstitution factor (volume of formulation after lyophilization: volume of formulation before lyophilization) may be 1:0.5 to 1:6. As described above, the lyophilizate according to the present disclosure may be reconstituted to provide an aqueous composition having an anti-VEGF hexameric antibody concentration of at least 40 mg/ml, for example, 40 mg/ml to 100 mg/ml, and the volume of reconstituent will be selected accordingly. If necessary, the reconstituted preparation may be diluted prior to administration to the patient at an appropriate timing to deliver the intended dosage. A typical reconstituent contains sterile water or buffer, optionally containing a preservative. Where a lyophilizate contains a buffer, the reconstituent may contain an additional buffer (which may be the same as or different from the buffer of lyophilizate) or may not contain a buffer (for example, water for injection (WFI) or physiological saline).

Examples of other excipients that may be included in the aqueous pharmaceutical composition according to the present disclosure are, but not limited to, antimicrobial agents, antioxidants, antistatic agents, lipids, for example, phospholipids or fatty acids, steroids, for example, cholesterol, protein excipients, for example, serum albumin (human serum albumin), recombinant human albumin, gelatin, casein, salt forming counterions, for example, sodium.

An aspect of the present disclosure provides an antibody composition including 90% by weight or more of the hexameric antibody based on the total weight of the composition.

In embodiments, the hexameric antibody may be included at 95 wt% or more.

The antibody composition may have a very high content of hexamers and may substantially not contain dimers, trimers, tetramers, and pentamers, except for monomers.

An aspect of the present disclosure provides a method of treating a disease or disorder mediated by VEGF, the method including administering the composition or the anti-VEGF antibody in a therapeutically effective amount to a subject or a patient.

The term "therapeutically effective amount" refers to an amount sufficient to produce a therapeutic effect when administered to a subject or a patient in need of treatment.

The term "treating or treatment" refers to treating a disease or a medical condition in a subject, for example, a mammal including a human, including:
(a) inhibiting the disease or medical condition, that is, slowing or stopping the progression of the disease or the medical condition in a subject; or
(b) alleviating a disease or a medical condition in a subject.

The term "subject" or "patient" refers to human and non-human mammals including, but not limited to, primates, rabbits, pigs, horses, dogs, cats, sheep and cattle. For example, a subject or a patient is a human being.

An aspect of the present disclosure provides a method of delivering an anti-VEGF antibody to a subject or a patient, the method including administering the pharmaceutical composition or the anti-VEGF hexameric antibody to the subject or the patient.

An aspect provides the use of the pharmaceutical composition or the anti-VEGF hexameric antibody in the preparation of a medicament for treating a disease or a disorder, mediated by VEGF, wherein the disease or the disorder may be an angiogenic disease, for example an angiogenic ocular disease, in particular, age-related macular degeneration.

An aspect provides a method of preparing a medicament for treating a disease or a disorder, mediated by VEGF, by using the pharmaceutical composition or the anti-VEGF hexameric antibody, the disease or the disorder may be an angiogenic disease, for example an angiogenic ocular disease, in particular, age-related macular degeneration.

Hereinafter, the present disclosure will be described in detail based on examples, but the examples are only to help understanding of the present disclosure and is not intended to limit the scope of the present disclosure in any way.

### <Preparation Example 1> Rabbit immunization and immune library preparation

Four rabbits (New Zealand White) were immunized with VEGF by intraperitoneal administration of VEGF165 (Sino biological, CA) four times. RNA was isolated from spleen and bone marrow extracts from four immunized rabbits. cDNA was synthesized using the GoScripts kit (Promega, USA) according to the manufacturer's instructions. The mRNA used at this time was 2 µg.

The variable region (VL and VH) genes of the rabbit antibody were amplified using the cDNA as a template. First, in order to amplify a variable region of a light chain (VL) of a rabbit antibody, PCR was performed using the cDNA, as a template, and 5'-specific primers set forth in SEQ ID NOS: 1, 2, 3, and 7 and 3'-specific primers set forth in SEQ ID NOS: 4, 5, 6, and 8, each in pairs, of the variable region of a light chain of the rabbit, so as to selectively amplify a rabbit antibody light chain gene. In order to amplify a variable region of a heavy chain (VH) of a rabbit antibody, PCR was performed using the cDNA, as a template, and 5'-specific primers set forth in SEQ ID NOS: 9, 10, 11, and 12 and 3'-specific primers set forth in SEQ ID NOS: 13 and 14, each in pairs, of the variable region of a heavy chain of the rabbit, so as to selectively amplify a rabbit antibody heavy chain gene. Finally, in order to amplify the scFv linker portion, PCR was performed using pComb3X-scFv DNA as a template and 5' specific primers set forth in SEQ ID NOS: 15, 16, and 17 and 3' specific primers set forth in SEQ ID NOS: 18, 19, 20, and 21, each in pairs, to selectively amplify the linker gene.

Extension PCR was performed using each VL, each linker, and each VH, amplified in the PCR, as a template. First, extension PCR of the VL gene was performed using 100 ng of the VL, linker, and VH genes amplified in the PCR, and the primer set forth in SEQ ID NO: 22 and the primer set forth in SEQ ID NO: 23. The conditions for PCR reaction are as follows: pre-denaturation was performed at 95 °C for 5 minutes, at 95 °C for 50 seconds, at 55 °C for 50 seconds, and at 72 °C for 1 minute; Ex-Taq DNA polymerase (Takara company) was used; and the test was repeatedly performed for 30 seconds.

The VL-Linker-VH (scFv) gene and the pComb3XSS vector obtained above were each digested with restriction enzyme Sfi I, purified, and the two DNA fragments were ligated and the enzyme was inactivated. After elution with distilled water using an XXPC DNA concentration column, the library DNA was transformed into *E. coli* ER2738 by electroporation.

Then, after culturing in SOC medium, the resultant was placed in a 2 L flask containing 500 ml SB + carbenicillin (50 µg/ml) and M13K07 helper phage (MOI: 1:20), and allowed to stand at 37 °C for 30 minutes, and kanamycin was added thereto in such an amount that the total volume reached 50 µg/ml, and incubated with shaking overnight at 30 °C. The next day, the culture medium was centrifuged to precipitate the cells, and only the library phages in the supernatant were collected.

### <Preparation Example 2> Phage library screening

Panning and screening were performed using the rabbit immune library phage prepared above. Bio-panning was performed three times with an Immunotube (Immunotube^{®}) coated with 5 µg/ml recombinant human VEGF fusion protein to select clones reactive to human VEGF. 96 phage clones were randomly selected from the colonies grown on the output plate and tested for reactivity to human VEGF by phage enzyme immunoassay.

DNA was sequenced for the final selected clones and classified into 6 scFv clones identified as having different complementarity determining region sequences. Thereafter, to confirm the VEGF binding ability, the final one type of 2-19 clone was selected through an enzyme immunoassay, and was confirmed to be a nucleotide sequences set forth in SEQ ID NO: 26 and SEQ ID NO: 27 which encode the amino acid sequence set forth in SEQ ID NO: 24 and SEQ ID NO: 25.

### <Preparation Example 3> Preparation of humanized antibody for rabbit anti-VEGF antibody

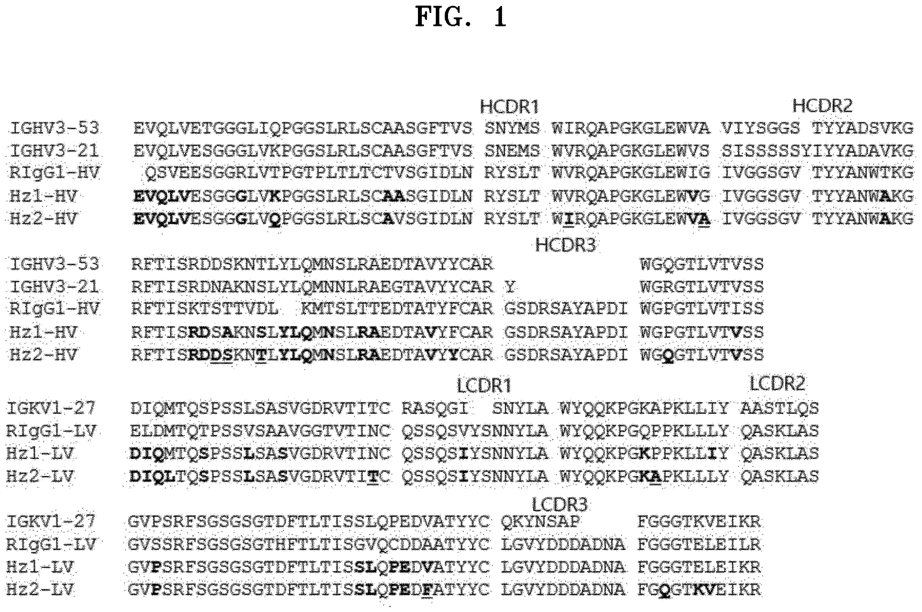

For humanized antibody design, human germ-line sequences similar to the variable region of a heavy chain and variable region of a light chain of the 2-19 clone were identified in IMGT/V-QUEST. In the case of the variable region of a heavy chain, IGHV3-21 and IGHV3-53 showed high similarity, and in the case of the light chain, IGKV1-27 showed high similarity. Based on this sequence, most of the framework parts were replaced with human sequences, and rabbit sequences and human sequences were introduced such that: heavy chain sequences that can affect antigen binding, and Kabat numbers D28 and D30 were substituted with rabbit sequences; and V24 was substituted with V or A, V37 was substituted with V or I, G49 was substituted with G or A, S73 was substituted with S or D, and F91 was substituted with F or Y, and T63 was designed to be substituted with A because glycosylation occurs in the CDR. The light chain was designed to introduce rabbit sequences and human sequences as follows: N or T for Kabat number N22, P or A for P43, L or I for L48, V or F for A83, and For Y for F91. FIG. 1 is a diagram in which the amino acids of rabbit is compared with the amino acids of a humanized anti-VEGF antibody. In FIG. 1, the bolded parts are the human sequence, and the underlined parts are the amino acid that contributed to the increase in solubility.

In order to convert the heavy and light chain portions to be converted into humanized sequences and the 2-19 scFv form into the whole IgG form, PCR (assembly PCR) combining synthetic oligo primers was performed using SEQ ID NOs: 26 and 27 as templates.

According to the instruction for the TAKARA Infusion kit for the animal expression vector pCEP6-WPRE (vector with Fc part inserted), a humanized antibody light chain expression vector and a heavy chain expression vector 2-19 were prepared in the same manner. Then, the obtained sequences were confirmed through DNA sequencing. SEQ ID NOs are as follows: Hz1 HV; SEQ ID NO: 28, Hz1 LV; SEQ ID NO: 29, Hz2 HV; SEQ ID NO: 30, and Hz2 LV; SEQ ID NO: 31.

### <Preparation Example 4> Preparation of mutant anti-VEGF antibody for increasing activity and improving hydrophilicity of Ranibizumab

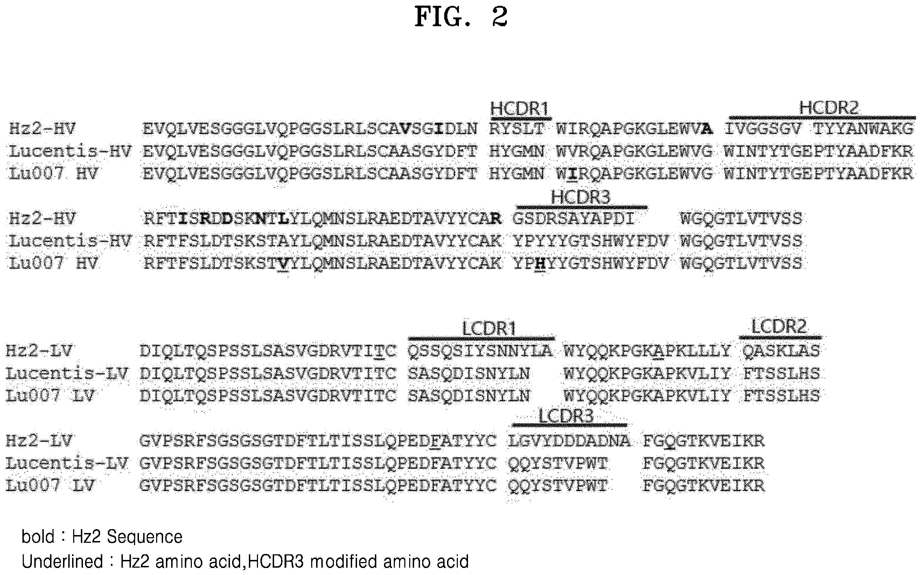

A phage library was prepared in order to convert from Ranibizumab (Lucentis) Fab form to whole antibody form, and to replace the framework sequence of the heavy chain variable region of the Hz2 antibody to increase activity and improve hydrophilicity. In the case of the framework part of the variable region of the heavy chain, a primer was prepared based on the Hz2 sequence to substitute Kabat numbers A24, V27, G49, F69, L71, T73, S76, A78, and K94 sequences with V, I, A, F, L, T, S, A, and R. As a result, 10 candidate groups were obtained, and 4 clones with good binding ability were selected therefrom: the variable region of Lu007 (SEQ ID NO: 32, SEQ ID NO: 33), the variable region of F8 (SEQ ID NO: 34, SEQ ID NO: 33), the variable region of E9 (SEQ ID NO: 35, SEQ ID NO: 33), and the variable regions of H2 (SEQ ID NO: 36, SEQ ID NO: 33). FIG. 2 shows a diagram in which the amino acid sequence of Lucentis is compared with the amino acid sequence of mutant Lucentis. In FIG. 2, the bold parts represents the Hz sequence, and the underlined parts represents the Hz2 amino acid and the HCDR3 modified amino acid.

### <Example 1> Preparation of anti-VEGF hexameric antibody

Antibody hexamers were prepared to increase the molecular weight of the antibody. The variable region of the hexamer included four types of antibodies and one type of receptor Fc fusion, including Avastin (Roche), Aflibercept (Regeneron), a humanized antibody prepared using the variable region of Hz1 (SEQ ID NOS: 28 and 29), a humanized antibody prepared using the variable region of Hz2 (SEQ ID NOS: 30 and 31), and the variable regions of Lu007 (SEQ ID NOS: 32 and 33). To facilitate the formation of a hexamer, in Hz1, the G44C mutation was introduced into HV and the G100C mutation was introduced into LV to prepare a hexamer. In order to facilitate the formation of hexamer for the antibody constant region Fc portion, mutations of Kabat number L309C, H310L, and E430H were introduced, and a mu-tailpiece (muTP, SEQ ID NO: 61) obtained from IgM was fused at the C-terminus of the Fc portion to prepare an antibody hexamer.

In order to decrease the binding of Fcγ receptor to the Fc portion, mutations of L234A, L235A, and P331G were each added to prepare an antibody (Hz2-F & Lu007-F).

Each gene was prepared by assembly PCR combining synthetic oligo primers, and the light chain expression vector and heavy chain expression vector of each antibody were prepared using the animal expression vector pCEP6-WPRE according to the instructions for the TAKARA Infusion kit. The obtained sequences were confirmed through DNA sequencing. The sequence number is as follows.
SEQ ID NO: 62: Aflibercept AAA0289;
SEQ ID NO: 63: Avastin AAA0292 heavy chain, SEQ ID NO: 64: Avastin AAA0292 light chain;
SEQ ID NO: 65: Avastin hexamer AAA0306 heavy chain;
SEQ ID NO: 66: Aflibercept hexamer AAA0308;
SEQ ID NO: 67: Hz1 hexamer AAA0322 heavy chain; SEQ ID NO: 68: Hz1 hexamer AAA0322 light chain;
SEQ ID NO: 69: Hz2-F hexamer AAA0589 heavy chain; SEQ ID NO: 70: Hz2-F hexamer AAA0589 light chain;
SEQ ID NO: 71: Lu007-F hexamer AAA0592 heavy chain, SEQ ID NO: 72: Lu007-F hexamer AAA0592 light chain;
SEQ ID NO: 73: Aflibercept CL kappa hexamer AAA0600.

### <Example 2> Antibody expression and purification

The expression of hexameric antibody was performed using the ExpiCHO-S system (Thermo). General culture of hamster ovarian cells ExpiCHO-S was performed according to the manufacturer's instructions. First, one day after subculture with ExpiCHO-S 2.5×10⁶ cells/ml, the number of cells was checked to confirm that the number was 5 ×10⁶ cells/ml to 6×10⁶ cells/ml, and the cell number was adjusted. Heavy chain and light chain expression vectors and ExpiFectamineCHO complexes were made. ExpiFectamineCHO/vector DNA complex was created and added to the culture medium at room temperature within 3 minutes, and then the flask was cultured in an incubator at 37 °C and under 8% CO₂ in a 125 rpm stirrer. After 18 to 22 hours, Enhancer and ExpiCHOFeed were slowly added and cultured in the incubator at 32 °C and under 5% CO₂ in the 125 rpm stirrer. ExpiCHOFeed was slowly added once more to D5 and cultured in the incubator at 32 °C and under 5% CO₂ in the 125 rpm stirrer. Twelve days thereafter, the culture medium was collected, centrifuged at 5000 rpm for 15 minutes at room temperature, and then allowed to pass through a 0.22 µm Amicon Ultra-15 (Millipore) filter to remove cell debris therefrom.

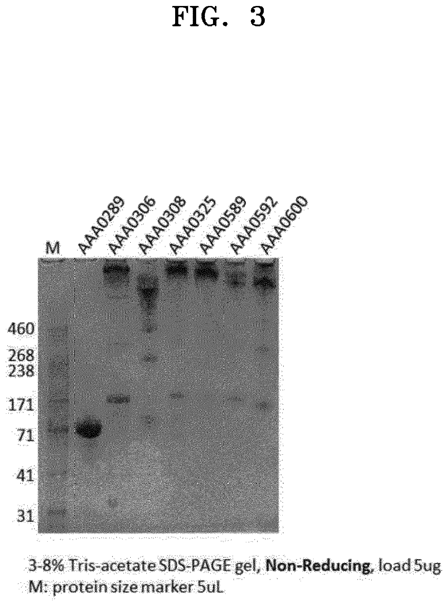

For purification, a column was equilibrated using an AKTA pure instrument and a Mabselectsure (GE) column and 1x PBS (pH 7.4) buffer, and then, the supernatant was loaded onto the column. Once the sample was entirely loaded, washing was performed thereon using 1x PBS (pH 7.4) buffer. When the UV280 value was equilibrated, the antibody was eluted using a 30 mM sodium acetate (pH 3.4) buffer. Immediately after elution, 1M Tris (pH 8.0) was added thereto to achieve neutralization. Immediately after neutralization, buffer exchange was performed with a buffer of 10 mM histidine, 135 mM NaCl, and pH 5.5. Once the purification was completed, the purification result was identified through SDS-PAGE. The results are shown in FIG. 3.

As shown in FIG. 3, it was confirmed that a monomer, a dimer, a trimer, a tetramer, a pentamer, and a hexamer were commonly formed in all hexamers. However, specifically, in the case of AAA0589, it was confirmed that the amount of hexamer was much higher than that of other hexamers, and there were almost no dimers, trimers, tetramers, and pentamers except for monomers.

### <Example 3> Perform ELSIA to confirm binding ability to VEGF

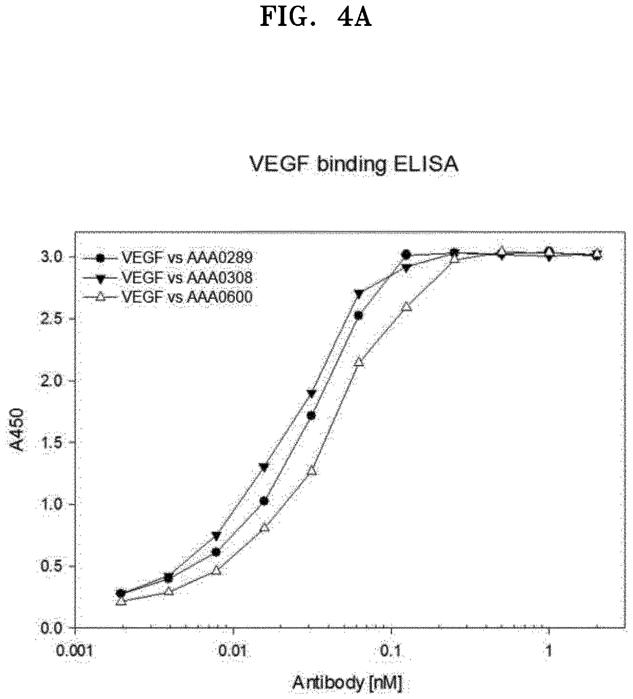

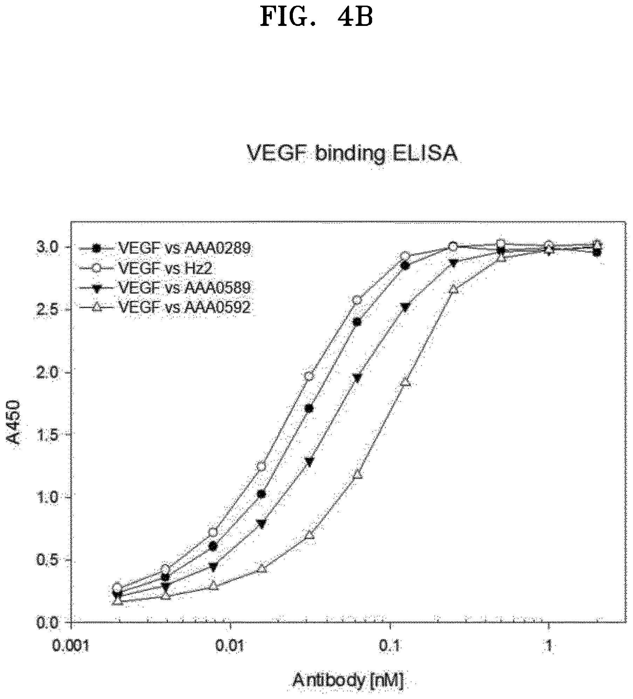

ELISA was performed to confirm the binding ability to VEGF. First, 100 µl of hVEGF was added to an immunoplate at a concentration of 500 ng/ml and caused to react at room temperature for 1 hour. 2% skim milk was dissolved in PBST, and then, 200 µl thereof was added onto the VEGF-coated wells and the reaction was caused to occur at room temperature for 2 hours. After the reaction, the well was washed three times using 300 µl of PBST, and then, Hz2, AAA0289, AAA0308, AAA0589, AAA0592, and AAA0600, which were purified above, were diluted starting from at a concentration of 100 ng/ml by two-fold to make a total of 11 samples. The samples were placed on the VEGF-coated well and the reaction was caused to occur at room temperature for 20 minutes. After the reaction, the well was washed three times using 300 µl of PBST, and then, anti-hlgG-Fc HRP (1:3000, Thermo) was added thereto at 100 µl/well and then reacted for 30 minutes. Then, the well was washed three times as described above, and the TMB (BioFx company) solution was added thereto at 100 µl/well to develop color at room temperature for 5 minutes. After the color development, 2N sulfuric acid was added at 50 µl/well to stop the color development, and the absorbance was measured at 450 nm, and results are shown in FIGS. 4A and 4B.

As shown in FIGS. 4A and 4B, it was confirmed that both monomers and hexamers were bound to VEGF.

### <Example 4> Determination of antibody affinity using Octet

Affinity for VEGF was determined as follows. Octet red96E was used, and human VEGF immobilized on the biosensor AR2G was interacted with AAA0308, AAA0589, AAA0592, and AAA0600 used as analytes, and binding and dissociation values thereof were compared and measured. As human VEGF, recombinant human VEGF165 (Sinobiological company) was used, and VEGF was immobilized on the AR2G sensor chip by an amine coupling method. At this time, the amount of VEGF used was 10 µg/ml, and the degree of coating was confirmed using a wavelength of 5 nm. In order to confirm the reactivity according to each concentration, in the case of AAA0589 and AAA0592, the antibody concentration was 4, 2, 1, 0.5, 0.25, 0.125, and 0.0625 nM, and in the case of AAA0600, antibody concentration was 0.6, 0.25, 0.125, and 0.0625 nM.

During measurement, the rotation speed was set to 100, and the temperature was measured at 30 °C.

The concentration of the sample was adjusted using a kinetic buffer (Molecular Device Co.). The antibody solution of each concentration was used as a binding phase for 5 minutes, and then switched to a kinetic buffer to form a dissociation phase for 30 minutes. Measurement values for each antibody are shown in Table 1. As a result, it was confirmed that AAA0589 and AAA0592 showed very good affinity of 1 pM or less, and AAA0600 also showed excellent affinity at the level of 2 pM.

**Table 1**

| Affinity measurement using Octet | | | |
|---|---|---|---|
| Sample | KD(M) | kon(1/Ms) | kdis(1/s) |
| AAA0308 | <1.0E-12 | 5.10E06 | <1.0E-07 |
| AAA0589 | <1.0E-12 | 2.08E+05 | <1.0E-07 |
| AAA0592 | <1.0E-12 | 6.66E+05 | <1.0E-07 |
| AAA0600 | 2.03E-12 | 6.35E+05 | 1.08E-05 |

### <Example 5> Comparison of calcium inhibition effect induced by VEGF in HUVEC cells

In order to compare the VEGF inhibitory effect of the antibody, the amount of the increase in calcium caused by VEGF in HUVEC cells was compared. This comparison test was performed using samples AAA0289, AAA0308, AAA0589, AAA0592, and AAA0600. EGM-2 HUVEC (LONZA, C2519AS) cells maintained between passages 2 and 7 in EBM-2 (Lonza-Clonetics, Walkersville, MD) supplemented with SingleOuots (Lonza-Clonetics, Walkersville, MD, #CC-4176) were used. The cells were dispensed at 30,000 cells/well on a black 96-well plate (NUNC, 165305) on which 0.1% collagen was coated. One day after the culture, calcium analysis was performed.

No purified material was added, or 0.078125, 0.15625, 0.3125, 0.625, 1.25, 2.5, 5, 10, or 20 nM antibodies were mixed with 1.5 nM VEGF165 (R&D systems, 293-VE-500), and then, incubated for 30 minutes at room temperature to induce the antibody-antigen bond.

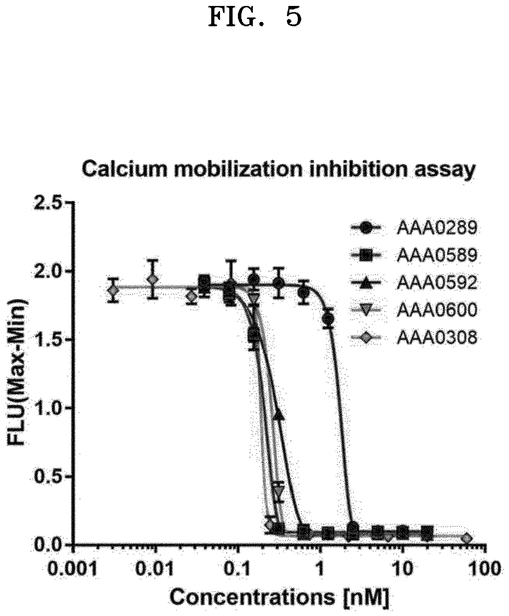

To measure the amount of intracellular calcium, a calcium assay kit (BD, #640176) was used according to the manufacturer's instructions, and a calcium indicator dye was added to each well, followed by incubation at 37 °C for 1 hour. The mixture treatment and the measurement of the amount of calcium in HUVEC cells were performed using a Flexstation (Flexstation III ROM v2.1.28, Molecular Devices). Fluorescence (excitation = 485 nm; emission = 525 nm) obtained from calcium indicator dye after 50 µl of treatment product was applied to a cell plate by using an automatic machinery, was recorded every 3 seconds over a period of 150 seconds. The results are shown in FIG. 5.

As shown in FIG. 5, in terms of the number of moles, it was confirmed that the IC₅₀ value of the hexamer and the IC₅₀ value of the commercially available AAA0289 (aflibercept) were 0.2 nM and 2.0 nM, respectively. That is, the IC₅₀ value of the hexamer was 10 times or more that of AAA0289. However, it was confirmed that there was little difference in IC₅₀ values of the hexamers.

### <Example 6> Concentration and aggregate of hexameric antibody and analysis of hexamer content

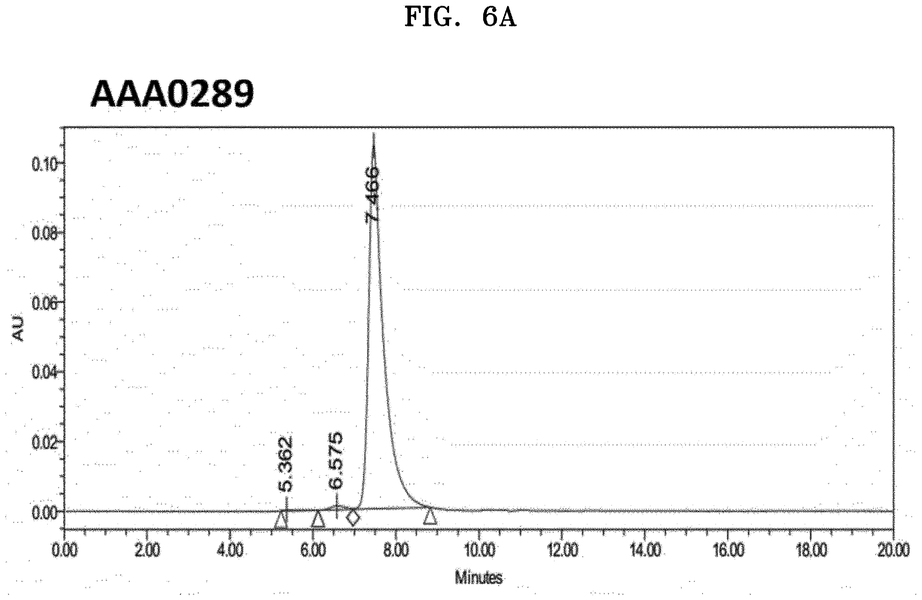

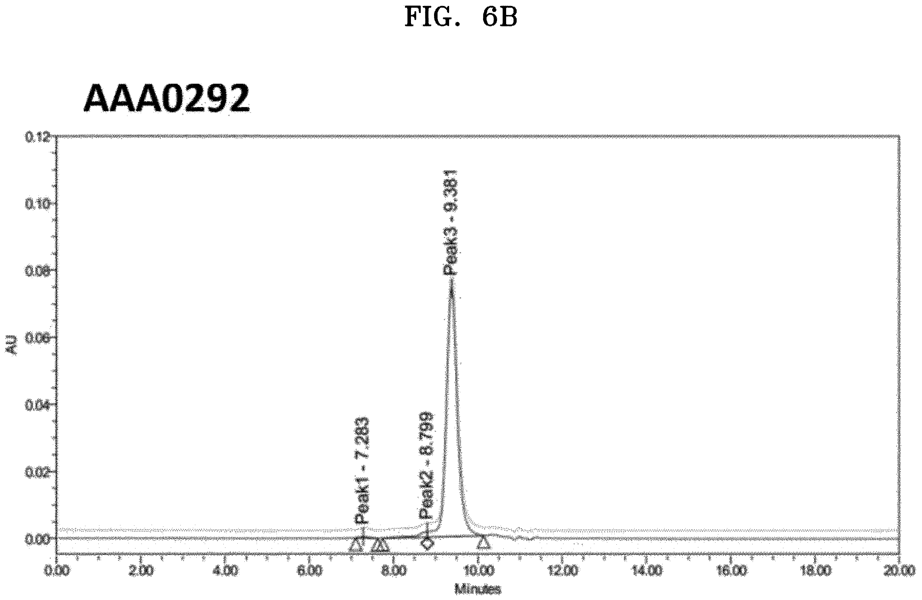

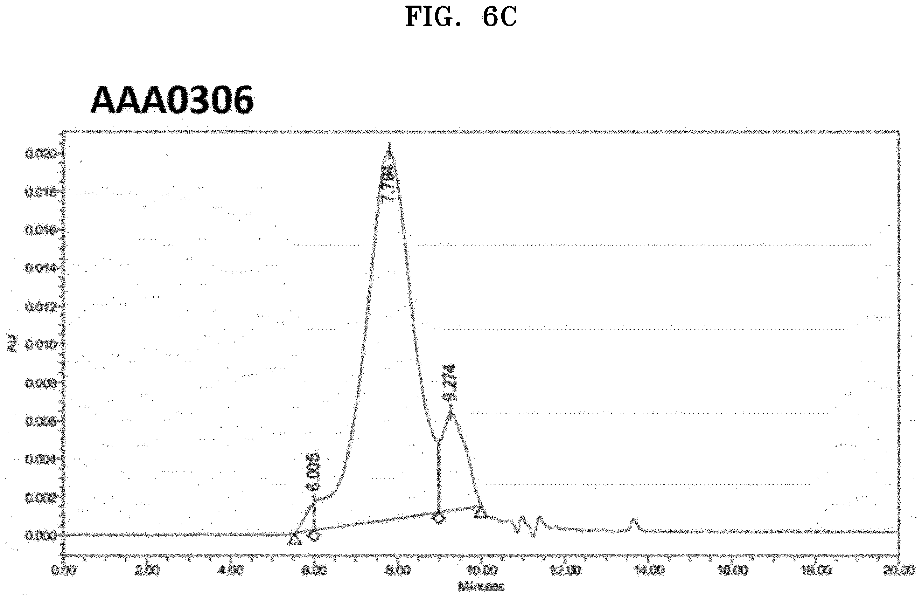

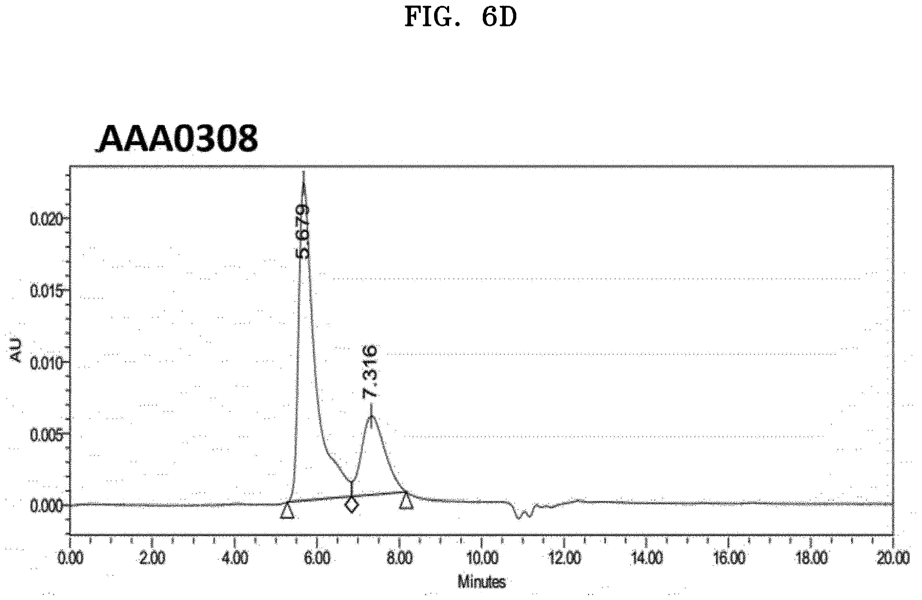

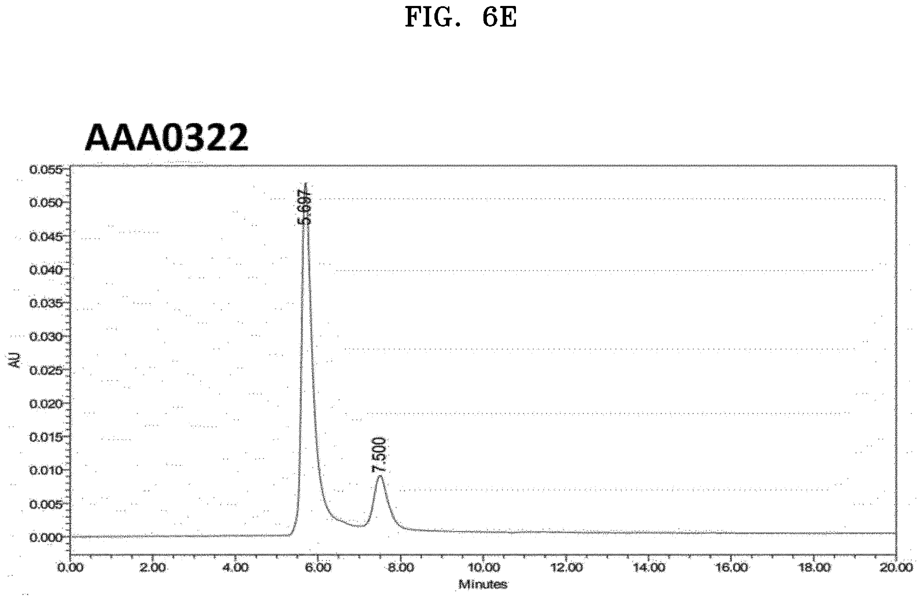

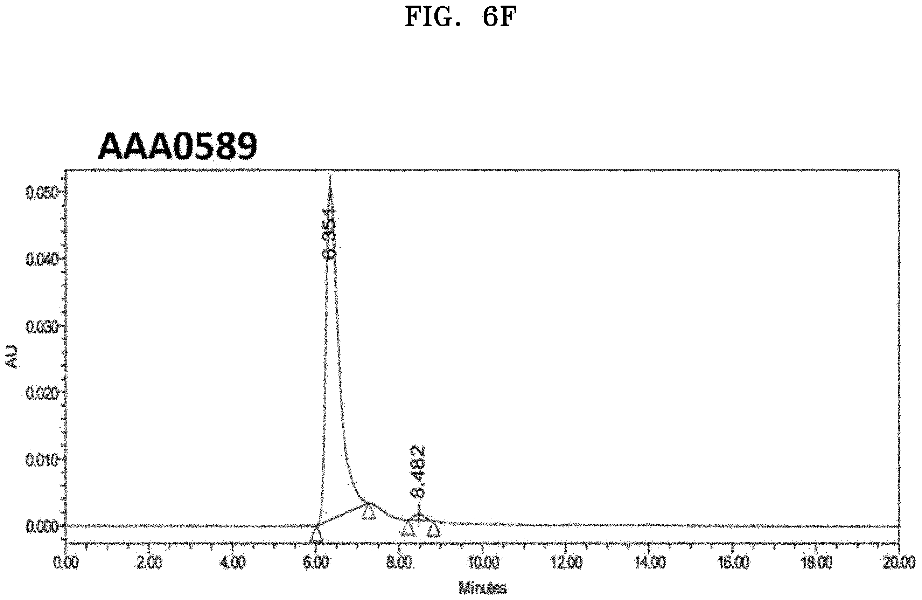

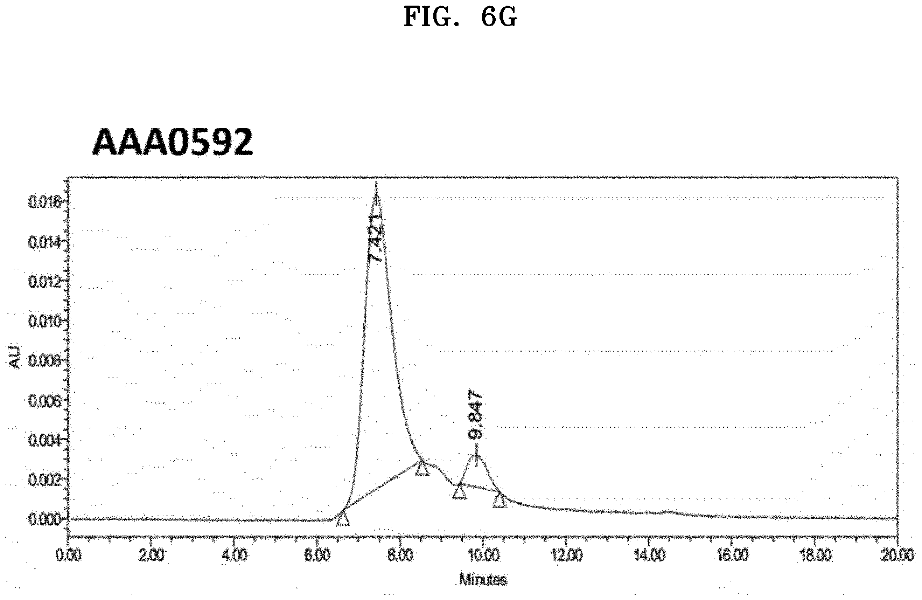

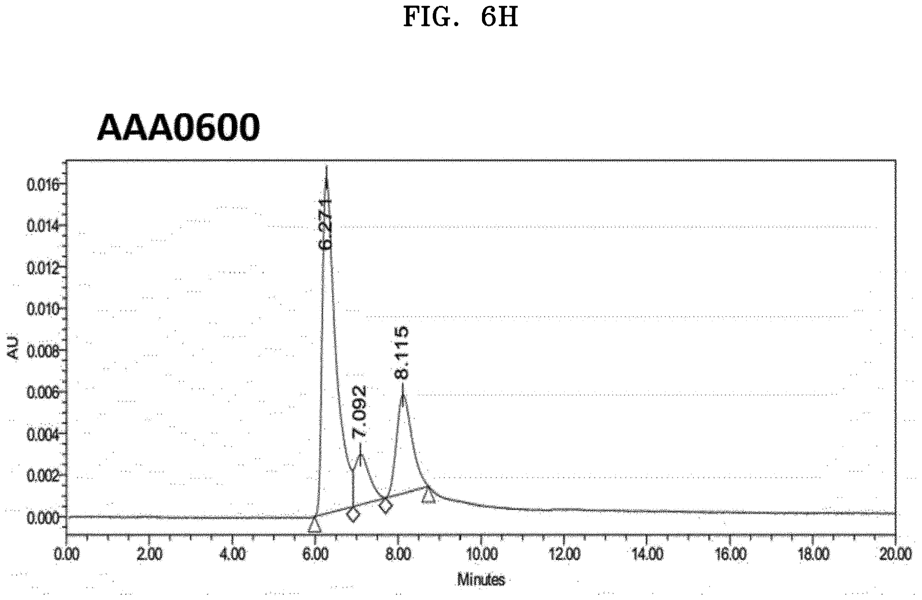

For the intraocular animal test, a protein concentration of at least 40 mg/ml was required. Accordingly, concentration and aggregate measurement tests were conducted. Concentration of the purified sample was performed using an ultrafiltration membrane, and after completion of the concentration, first, the formation of aggregates was confirmed using a gel filtration chromatography method. The conditions are as follows. A SEC-3 300A, 7.8 × 300 mm (Agilent) column was used in a Waters Alliance HPLC instrument, and 1 × PBS pH 7.4 buffer was used as the mobile phase, and the test was conducted at a flow rate of 1.0 ml/min. Results are shown in FIGS. 6A, 6B, 6C, 6D, 6E, 6F, 6G, and 6H. In terms of hexamer content, it was confirmed that AAA0322 and AAA0589 formed the greatest amount of hexamer.

During the concentration process, in the case of Avastin hexamer AAA0306 and Hz1 hexamer AAA0325, when concentrated at 10 mg/ml or more, many aggregates were generated, and in the case of Lu007 hexamer AAA0592, after concentrated at 40 mg/ml, the hexamer was continuously unstable and aggregates were formed.

In addition, in the case of AAA0308, AAA0589, and AAA0600, it was visually confirmed that no aggregates were formed when concentrated at a concentration of 40 mg/ml, and, in the case of AAA0589, even when concentrated at a concentration of 100 mg/ml, no aggregates were formed.

### <Example 7> Thermal stability evaluation through Tm value measurement

It is desirable to have high thermal stability in order to formulate a stable antibody formulation. As a method for evaluating stability, the evaluation of the middle temperature of heat denaturation (Tₘ value) was performed. In general, it is preferable that the middle temperature of heat denaturation is high. The thermal stability of each antibody was measured by protein thermal shift (PTS) analysis, which is performed by measuring the fluorescence of the dye according to an increase in temperature caused by the introduction of a dye having binding force with the hydrophobic portion when the hydrophobic portion located inside the protein is exposed in the folded state of the protein. The PTS assay was performed using the protein thermal shift dye kit (Catalog No. 4461146, Applied Biosystems) according to the manufacturer's method. Specifically, 5 µg of each antibody was mixed with protein thermal shift dye and buffer in such an amount that the total amount was 20 µl, and then, in RT-PCR (C1000 thermal cycler equipped with a CFX96 optical reaction module, Bio-Rad), the ROX signal was monitored from 20 °C to 95 °C while increasing the temperature by 1 °C per 60 seconds. Based on the melting curve, the melting temperature (Tₘ) of each antibody was determined.

The melting points of the antibodies are shown in Table 2. As a result, it was confirmed that AAA0589 and AAA0592 showed good thermal stability, and it was confirmed that, in general, the Aflibercept-based hexamer AAA0308 and AAA0600 showed low thermal stability. The difference between the thermal stability thereof and that of antibody-based hexamers AAA0589 and AAA0592 was more than 10 °C.

**Table 2**

| Thermal stability test using thermal shift analysis | |
|---|---|
| Sample | Tₘ(°C) |
| AAA0289 | 65 |
| AAA0308 | 63 |
| AAA0589 | 73 |
| AAA0592 | 75 |
| AAA0600 | 59 |

### <Example 8> Evaluation of binding to FcγR I, II, and III for AAA0589 and AAA0592

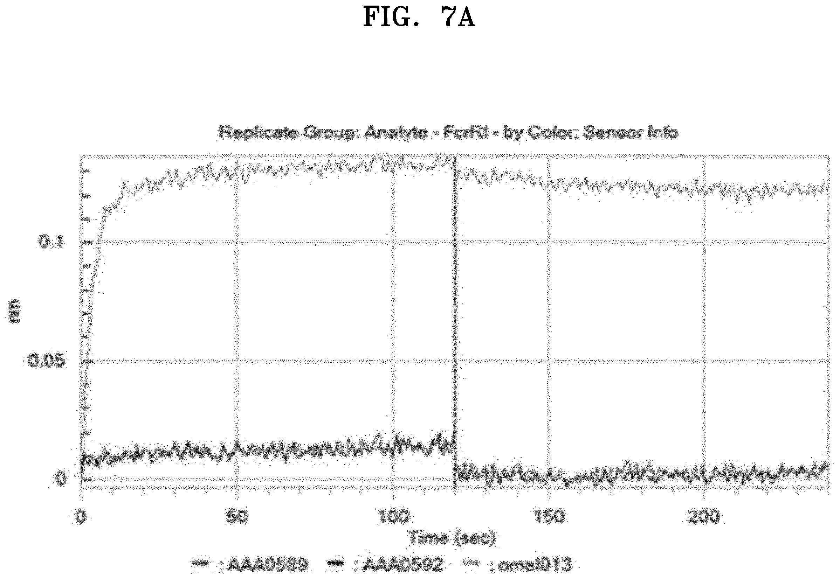

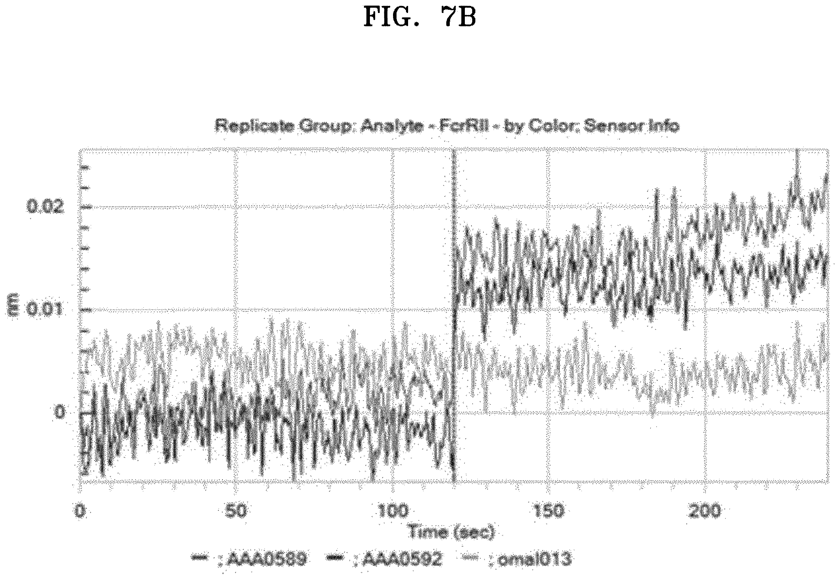

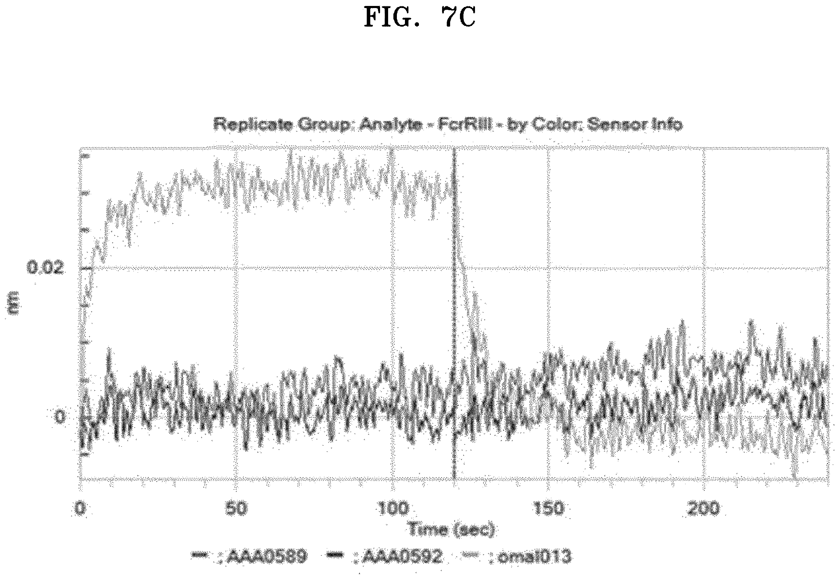

Binding evaluation for FcγR I, II, and III was performed as follows. Octet red96E was used, and 100 mM FcγRI(R&D system company, 1257-Fc), 100 mM FcγRII(R&D system company, 1597-CF), and 100 mM FcyRIII(R&D system company, 1257-Fc) were each loaded on the anti-pentia his biosensor, and a signal of about 5 nm was confirmed. In order to identify the reactivity of AAA0589 and AAA0592 to FcγRI, FcγRII, and FcγRIII, binding force was measured using an antibody concentration of 100 nM as an analyte. During measurement, rpm was set to 100, and the temperature was measured at 30 °C. Samples were diluted using kinetic buffer (Molecular Device Co.). The antibody solution of each concentration was used as an association phase for 2 minutes, and then converted to a kinetic buffer to form a dissociation phase for 2 minutes. The binding force between the Fcγ receptor and the antibody is shown in FIGS. 7A, 7B, and 7C.

As shown in FIGS. 7A, 7B, and 7C, in the case of the positive control group (Omal013; heavy chain: SEQ ID NO: 76, light chain: SEQ ID NO: 70), it seems to bind to the Fcγ receptor normally, and in the case of AAA0589 and AAA0592, of which binding force with the Fcγ receptor was reduced, the binding did not occur, as expected.

### <Example 9> Assessment of binding of AAA0589 and AAA0592 to FcRn

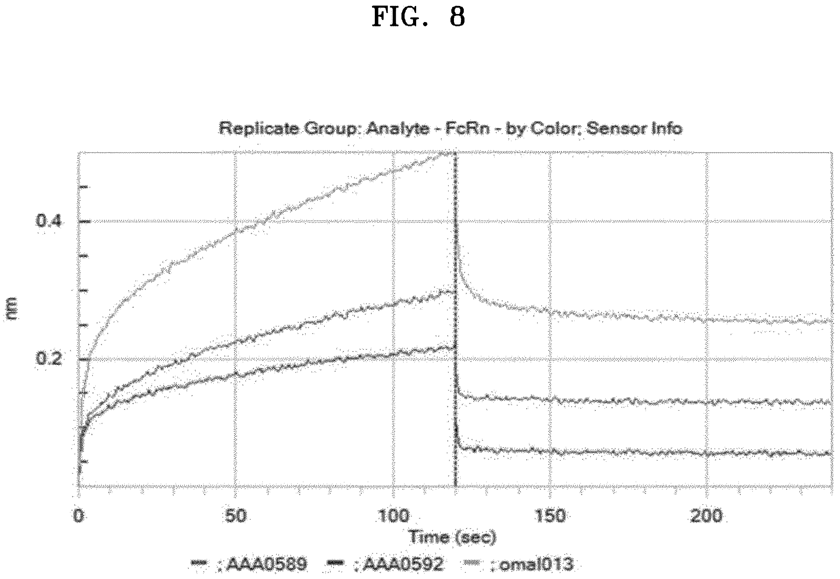

Evaluation of binding to FcRn (neonatal Fc receptor; R&D system company, 8639-Fc) was performed as follows. Octet red96E was used, and 10 µg/ml of positive control (Omal013), AAA0589, and AAA0592 antibodies were loaded onto the FAB2G (anti-Fab 2nd generation) biosensor, and a signal of about 5 nm was confirmed. In order to confirm the reactivity to FcRn, 400 nM FcRn was diluted with FcRn binding buffer (100 mM sodium phosphate, 150 mM NaCl, 0.05% Tween20, pH 6.0) and used as an analyte, and the rpm was set to 100 during measurement, and the temperature was measured at 30 °C. Samples were diluted using a FcRn binding buffer. A solution
of 400 mM FcRn was used as an association phase for 2 minutes, and then switched to FcRn binding buffer to form a dissociation phase for 2 minutes. The binding force between FcRn and the antibody is shown in FIG. 8.

As shown in FIG. 8, it can be seen that the positive control group (Omal013) binds to FcRn normally, and the binding ability of hexamers AAA0589 and AAA0592 was reduced compared to the positive control group.

### <Example 10> Rabbit intraocular residual amount measurement test after hexamer administration

The effect of extending the intraocular half-life of the hexameric antibody was confirmed by administering the hexameric antibody into the rabbit vitreous body and measuring the concentration of the hexameric antibody in the anterior chamber, the vitreous body, the retina and the choroid at 7 different time points from the day of administration to 30 days. The test method is as follows.

A New Zealand white rabbit weighing about 2 kg was anesthetized with a mixture of ketamine hydrochloride (35 mg/kg) and lumpoon (xylazine, 5 mg/kg) through intramuscular injection, and then, ocular anesthesia was performed by dropping 1 to 2 drops of proparacaine, which is an eye anesthetic, on the eyes. Iridodilator was administered if necessary. The conjunctival part was disinfected with povidone-iodine, and then, the eyes were exposed with an eye speculum, and the candidate protein and the positive control protein were administered at an amount of 2 mg/eye into the eyes through intravitreal injection by using a Hamilton syringe at a distance of 1 mm or more from the surgical limbus. The administration volume was 50 µl and care was taken not to increase intraocular pressure, and 3 animals per group (a total of 6 left/right eyes) were used for sample administration. After administration, rabbits were euthanized at 7 predetermined time points (0.3, 1, 4, 7, 14, 21, and 30 days), and the rabbits were euthanized and the aqueous humor was withdrawn using a syringe. After extracting the eyeball, the vitreous body, the retina, and the choroid were separated, and each zirconium bead was added thereto and the tissues were ground using a tissue grinder (Precellys 24) to make a solution. The concentration of the candidate substance and the control group were confirmed by immunoassay (ELISA). Quantification using ELISA was performed as follows. First, 100 µl of hVEGF was added to an immunoplate at a concentration of 500 ng/ml and reacted overnight at 4 °C. 2% BSA was dissolved in PBST, and then, 200 µl of each was added to the VEGF-coated wells and reacted at room temperature for 1 hour. After the reaction, the wells were washed 3 times with 300 µl of PBST, and then the purified Standard and QC samples were prepared, put into the VEGF-coated wells, and reacted at room temperature for 1 hour. Dilution of each sample was performed as follows. As for the concentrations of the standards, in the case of Aflibercept AAA0289, which was prepared in-house, a total of 11 concentrations were prepared: 0.22 ng/m£, 0.36 ng/m£, 0.58 ng/m£, 0.93 ng/m£, 1.49 ng/m£, 2.3 ng/mℓ, 3.8 ng/mf, 6.1 ng/m£, 9.7 ng/m£, 15.6 ng/mf, and 25 ng/m£; and in the case of hexameric antibody AAA0589, a total of 11 concentrations were prepared: 0.36 ng/m£, 0.58 ng/m£, 0.93 ng/m£, 1.49 ng/m£, 2.3 ng/mℓ, 3.8 ng/mf, 6.1 ng/m£, 9.7 ng/m£, 15.6 ng/mf, 25 ng/mf, and 40 ng/m£. As for QC samples, in the case of AAA0289, a total of 5 concentrations were prepared: 0.42 ng/m£, 0.83 ng/m£, 2.5 ng/m£, 7.5 ng/m£, 15 ng/m£; and in the case of AAA0589, a total of 5 concentrations were prepared: 0.83 ng/m£, 1.67 ng/m£, 5.0 ng/m£, 15 ng/mf, and 30 ng/m£. Among the analysis samples, the vitreous body sample was first diluted 16,000 times and then diluted again 2 times to prepare 3 diluted samples up to a final 64,000 times. In the case of the aqueous humor, retina and choroid samples, 4 diluted samples were prepared by diluting from 1,600 times by 2 times, up to 64,000 times. After reacting for 1 hour, the wells were washed 3 times with 300 µl of PBST, and anti-hlgG-Fc horseradish peroxidase (Thermo Fisher Scientific) was diluted at 1:20,000 for AAA0589 and 1:40,000 for AAA0289 at 100 µl/well. After reacting for 1 hour and washing three times as above, 100 µl/well of tetramethylbenzidine (BioFx) solution was added and color was developed at room temperature for 10 minutes. When the color development was finished, 50 µl/well of 2M sulfuric acid was added thereto to stop the color development, and the absorbance was measured at 450 nm using Spectramax i3 (Molecular devices), and SoftmaxPro6.4 version was used, and the quantification was determined by a curve titration using five parameters.
Results are shown in Tables 3, 4, 5 and 6.

As shown in Table 4, it was confirmed that the 900 kDa hexameric antibody migrated well to the retina and the choroid, and on average, the amount of antibody remaining in the vitreous body and retina/choroid of hexameric antibodies was equal to or higher than that of monomeric antibodies, and the equivalent or higher concentrations were maintained up to 30 days after administration. In particular, the half-life of AAA0589 in the vitreous body was increased by 3.3 times on the 14th day of administration, and by 1.6 times on the 30th day. Accordingly, it was confirmed that AAA0589 had better half-life extension effect than AAA0289. In the half-life calculation, the total amount of vitreous body was calculated as 1.25 ml.

**Table 3**

| Result of measuring the amount of antibody in the vitreous body, the retina and the choroid of AAA0289 monomer | | | | | | |
|---|---|---|---|---|---|---|
| Day | Vitreous body (µg/ml) | | Aqueous humor (µg/ml) | | Retina and choroid (µg/ml) | |
| | Average | Standard Deviation | Average | Standard Deviation | Average | Standard Deviation |
| 0 | 1593.9 | 269.0 | 29.7 | 33.7 | 21.3 | 8.0 |
| 0.3 | 1300.4 | 248.5 | 33.3 | 44.1 | 14.6 | 7.0 |
| 1.0 | 1121.8 | 175.0 | 77.1 | 33.5 | 14.7 | 6.5 |
| 4.0 | 773.0 | 92.6 | 53.0 | 9.1 | 12.8 | 2.8 |
| 7.0 | 594.4 | 48.0 | 37.6 | 10.5 | 7.8 | 3.8 |
| 14.0 | 131.3 | 19.0 | 8.3 | 2.4 | 3.4 | 1.8 |
| 21.0 | 42.3 | 40.9 | 5.6 | 0.9 | 0.9 | 0.3 |
| 30.0 | 13.8 | 5.4 | 0.2 | 0.1 | 0.1 | 0.1 |

**Table 4**

| Result of antibody amount measurement in vitreous body, retina and choroid of AAA0589 hexameric antibody | | | | | | |
|---|---|---|---|---|---|---|
| Day | Vitreous body (µg/ml) | | Aqueous humor (µg/ml) | | Retina and choroid (µg/ml) | |
| | Average | Standard Deviation | Average | Standard Deviation | Average | Standard Deviation |
| 0 | 1739.0 | 287.0 | 27.9 | 37.6 | 124.9 | 92.6 |
| 0.3 | 1607.9 | 243.7 | 37.7 | 36.2 | 23.7 | 17.7 |
| 1.0 | 1628.2 | 222.9 | 61.5 | 43.4 | 18.3 | 12.3 |
| 4.0 | 1334.3 | 200.1 | 61.7 | 31.5 | 53.6 | 28.7 |
| 7.0 | 1431.6 | 90.0 | 38.0 | 18.8 | 17.0 | 8.9 |
| 14.0 | 797.6 | 109.1 | 16.9 | 6.8 | 10.4 | 12.1 |
| 21.0 | 278.5 | 154.1 | 10.7 | 1.2 | 1.5 | 1.7 |
| 30.0 | 78.3 | 67.7 | 1.8 | 0.4 | 2.5 | 1.9 |

**Table 5**

| Results of half-life measurement after 14 days of rabbit intraocular administration | | | | | |
|---|---|---|---|---|---|
| Sample | Eye tissue | Remaining amount after 14 days (µg) | Dose (µg) | Half-life (T1/2), days | Increase rate with respect to AAA289 |
| AAA028 9 | Vitreous body | 131.3 | 2000.0 | 4.2 | 1.0 |
| AAA028 9 | Aqueous humor | 8.3 | 2000.0 | 4.0 | 1.0 |
| AAA028 9 | Retina and choroid | 3.4 | 2000.0 | 5.3 | 1.0 |
| AAA058 9 | Vitreous body | 797.6 | 2000.0 | 13.9 | 3.3 |
| AAA058 9 | Aqueous humor | 16.9 | 2000.0 | 6.5 | 1.6 |
| AAA058 9 | Retina and choroid | 10.4 | 2000.0 | 4.7 | 0.9 |

**Table 6**

| Results of half-life measurement after 30 days of rabbit intraocular administration | | | | | |
|---|---|---|---|---|---|
| Sample | Eye tissue | Remaining amount after 30 days (µg) | Dose (µg) | Half-life (T1/2), days | Increase rate with respect to AAA289 |
| AAA028 9 | Vitreous body | 13.8 | 2000.0 | 3.9 | 1.0 |
| AAA028 9 | Aqueous humor | 0.2 | 2000.0 | 5.1 | 1.0 |
| AAA028 9 | Retina and choroid | 0.1 | 2000.0 | 4.1 | 1.0 |
| AAA058 9 | Vitreous body | 78.3 | 2000.0 | 6.4 | 1.6 |
| AAA058 9 | Aqueous humor | 1.8 | 2000.0 | 4.6 | 0.9 |
| AAA058 9 | Retina and choroid | 2.5 | 2000.0 | 5.2 | 1.3 |

### <Example 11> Prediction of human pharmacokinetics derived from rabbit pharmacokinetic tests of hexameric antibodies

Rabbit pharmacokinetic data of the hexameric antibody AAA0589 and competitor KSI-301 (Kodiac Sciences) were used to predict human pharmacokinetic data according to the interspecies scaling method, and at this time, the human vitreous body volume was assumed to be a physiological maximum volume of 4 ml. For prediction, rabbit intravitreal pharmacokinetic data of the hexamer of Example 11 and rabbit intravitreal pharmacokinetic data of KSI-301 (Kodiak Corporate presentation March 2020; https://ir.kodiak.com) were used. Phoenix WinNonlin software was used to calculate pharmacokinetic parameters, and mrgsolve (R package) software was used to predict human pharmacokinetics.

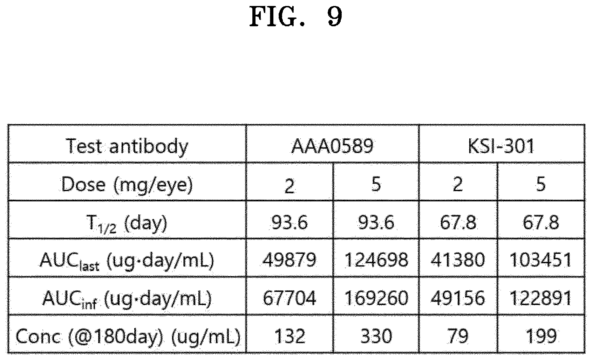

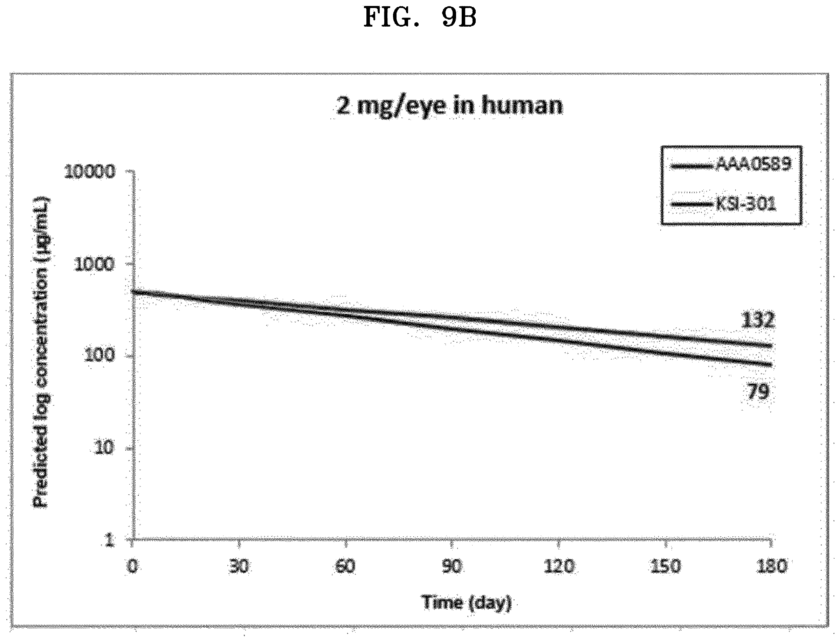

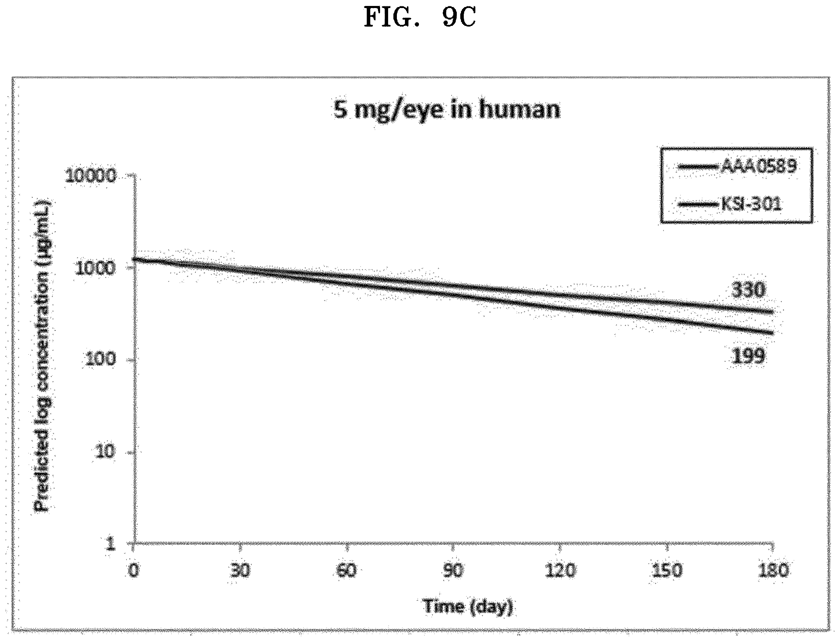

As shown in FIGS. 9A, 9B, and 9C, the predicted clearance (CLivt) in human aqueous humor of the hexameric antibody and KSI-301 were about 0.030 ml/day and 0.041 ml/day, respectively, indicating that the hexameric antibody was lost more slowly within the aqueous humor than KSI-301. In addition, the predicted half-lives in human aqueous humor were about 93.6 and about 67.8 days for the hexameric antibody and KSI-301, respectively, indicating that the hexameric antibody has a longer half-life in the aqueous humor than KSI-301. The predicted residual concentration of the drug in the aqueous humor after 6 months (180 days) after administration, was 132 µg/ml when the hexameric antibody was administered at 2 mg/eye and 199 µg/ml when KSI-301 was administered at 5 mg/eye. That is, the hexameric antibody remained at a concentration similar to that of KSI-301 in the aqueous humor even when a lower dose than that of KSI-301 was administered.

This is because the hexameric antibody has a lower aqueous humor clearance than KSI-301.

### <Example 12> Efficacy test of hexameric antibody using rat choroidal neovascularization model

In order to determine whether the hexameric antibody is effective in suppressing abnormal choroidal neovascularization, laser is irradiated on the eyes of brown-Norwegian rats to induce choroidal neovascularization (CNV), and then the hexameric antibody and a control substance Aflibercept were administered and efficacy was evaluated. The test method is as follows. Male 6-week-old brown-Norwegian rats were obtained and acclimated for 7 days. An iridodilator (Madriacil 1% eye drop, Alcon) was instilled into the animal's right eyeball, and then the animal to be photographed was anesthetized. Then, a laser (Elite, montane, USA) was irradiated to the eyeball under conditions of 532 nm, power of 100 mW, duration of 0.1 sec, and six points were made centering on the optic nerve. On the day of laser irradiation, while the animal was anesthetized, 10 µl of the test substance was intravitreally administered to the right eye of the animal using a syringe equipped with a 31 gauge needle. The groups administered were the vehicle control group, the positive control group (administered with Aflibercept at 400 µg/eye), a group administered with the hexameric antibody AAA0589 at 400 µg/eye, and a group administered with the hexameric antibody AAA0589 at 1000 µg/eye. Body weight was measured once a week from the start of test substance administration. In addition, after 0, 3, 7, 10 and 14 days from the test substance administration date, iridodilator was instilled into the left eye of the animal, and then the animal was anesthetized, and about 0.5 ml of 2% fluorescent sodium salt (Sigma-Aldrich, USA) was injected through the tail vein. Eyeballs were photographed within about 2 minutes using a fundus camera (TRC-50IX, TOPCON, Japan). Retinal CNV confirmation and drug efficacy evaluation were performed using retinal fluorescence fundus photography. For image analysis, the fluorescence intensity of the irradiated area was analyzed using ImageJ software (NIH, Bethesda, MD). On the 14th day after administration of the test substance, the animals were anesthetized and then exsanguinated/killed. The right eye was removed, a hole was made in the center of the cornea using a syringe, and was fixed in 10% neutral buffered formalin solution. The fixed right eye was stained with Isolectin B4 (Isolectin GS IB₄, Alexa Fluor^{™} 488 conjugate, Invitrogen^{™}, Lot 278700) to calculate the CNV area (*µ*m2) as data.

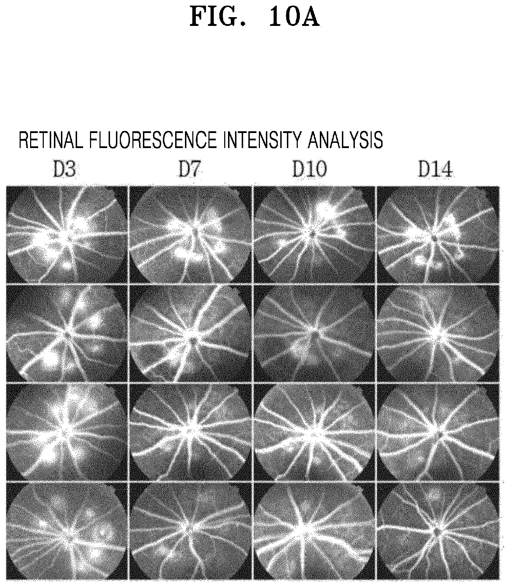

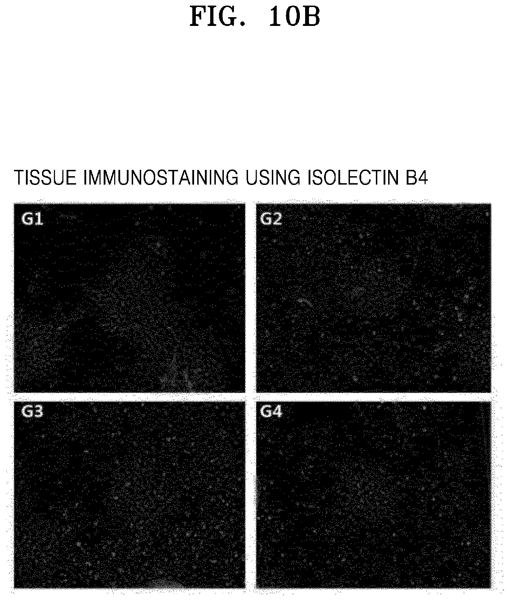

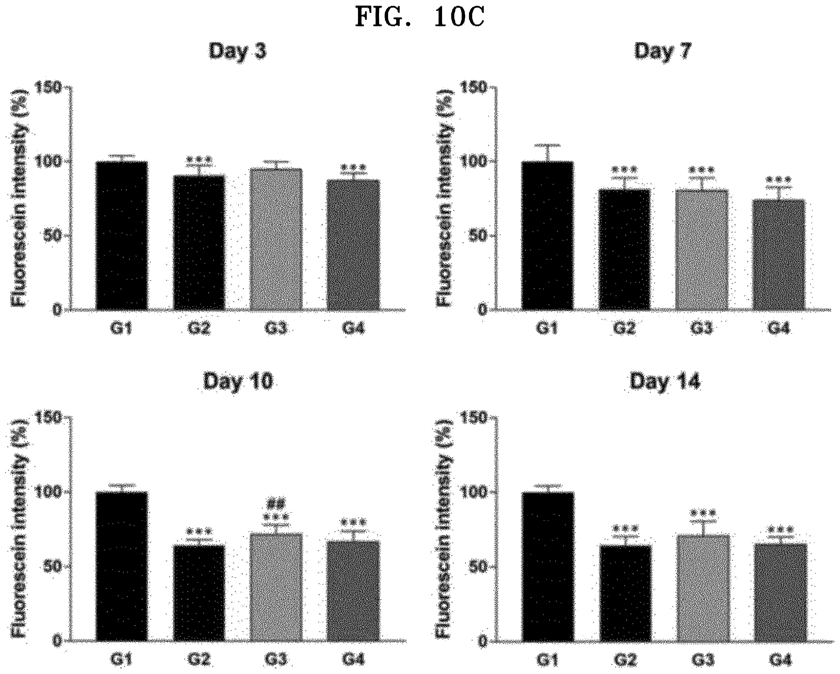

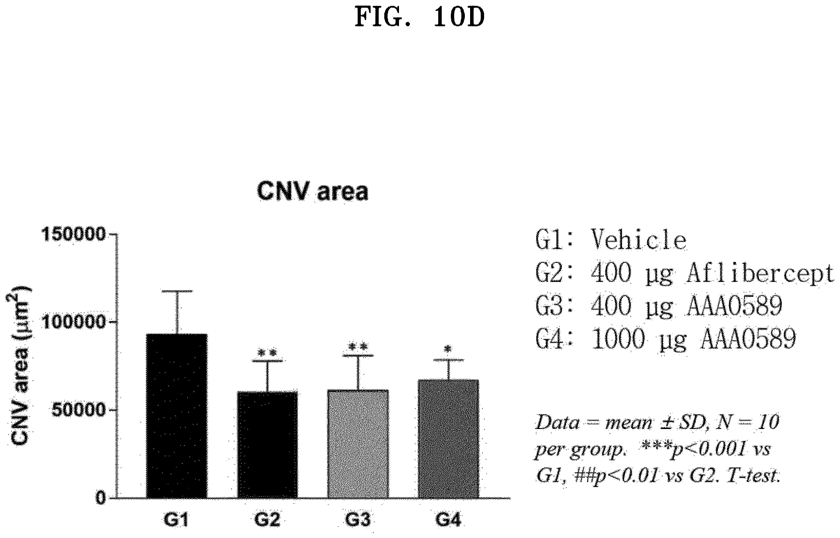

As shown in FIGS. 10A, 10B, 10C, and 10D, as a result of retinal fluorescence intensity analysis, the retinal fluorescence intensity level of the hexameric antibody 400 µg/eye administration group was statistically significant from the 7th day to the 14th day after administration of the test substance compared to the solvent control group. The retinal fluorescence intensity level of the hexameric antibody 1000 µg/eye administration group was also significantly lower than that of the solvent control group from the 3rd day to the 14th day after administration of the test substance.

The level of retinal fluorescence intensity was decreased in a dose-related manner in the test substance AAA0589 administration group.

This result may be obtained due to the action of the test substance. As a result of eyeball removal and analysis at the end of the test, the coroidal vascularization area level in the hexameric antibody-administered group was statistically significantly lower than that of the control group. In conclusion, intravitreal administration of hexameric antibodies is thought to be helpful in suppressing choroidal neovascularization in a choroidal neovascularization model using laser irradiation-induced brown Norwegian rats.

### <Example 13> Monkey retina penetration test of anti-VEGF hexameric antibody

In order to evaluate whether the anti-VEGF hexameric antibody has penetration ability in the retina of apes, a single intravitreal administration test was performed on cynomolgus monkeys, and the degree of penetration of the drug into the retina was evaluated using a confocal microscope for the autopsied individuals. Five male monkeys aged 28 to 32 months and weighing in the range of 2.3 to 2.6 kg were properly reared in the animal room before the test was started, and had an acclimatization period of more than one week. Before the test was started, the body weight was measured to select animals that were not affected by the test, and group separation was carried out into 1 group by 1 animal. 5 mg of a hexameric antibody and 1.25 mg of a control substance Avastin were each administered into the vitreous body at 50 µl per eye.

Autopsies were performed for each group on the 1st, 7th, and 14th days after administration of the test substance. For autopsy, hyperanesthetization was induced using an injectable anesthetize agent, and then the eyeballs were removed by transblepharoptosis. The extracted eyeballs were fixed in Davidson's solution, and fabricated into paraffin blocks, and sectioned through general tissue treatment processes such as trimming, dehydration, and paraffin infiltration. The Cys3 AffiniPure F(ab')₂ Fragment Donkey Anti-Human IgG (H+L) antibody was subjected, on a trimmed tissue fragment, to immunofluorescence staining at 1:500, and then, the degree of permeability within the retina was evaluated by using a confocal microscopy (LSM880, Carls Zeiss, Germany).

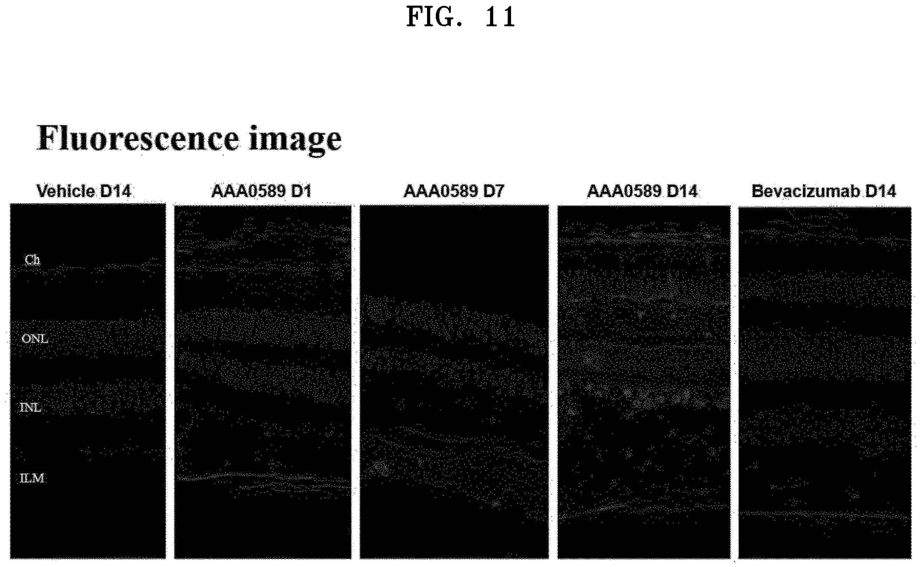

As shown in FIG. 11, as a result of confocal microscopy, it can be seen that, in the eyes which were stained by immunofluorescence using Cys3 AffiniPure F(ab')₂ Fragment Donkey Anti-Human IgG (H+L) antibody, one day after administration, positive staining for the test substance was observed in the inner limiting membrane of the retina and blood vessels of the choroid. 7 days after administration, positive staining for the test substance was observed in the nerve fiber layer of the retina. 14 days of administration, it was confirmed that the test substance penetrated between the inner nuclear layer and the outer nuclear layer of the retina. In the case of the Avastin-administered group, positive staining was observed in the retinal inner limiting membrane and choroidal blood vessels 14 days after administration. No dead subjects were observed during the entire test period, and as a result of histopathological examination, inflammation of the conjunctiva and retina was observed in all test substance administration groups, but these changes are background lesions that can be observed in the corresponding animal species due to intravitreal administration. In conclusion, it is judged that the anti-VEGF hexameric antibody can penetrate the retina of apes within 14 days of administration.

### <Example 14> Analytical ultracentrifuge analysis for confirmation of hexamer formation

Analytical ultracentrifuge analysis was performed to identify the molecular weight of the hexamer. The analytical ultracentrifuge is a technology that combines the performance of a centrifuge to sediment particles and the function of an optical module to detect sedimentation over time, and can predict the molecular weight, shape, and type of a sample. The method is as follows. The cell was completed by assembling the charcoal-filled EPON center piece and other parts, and 400 µl buffer and 390 µl sample were injected into each hole. Samples were prepared by diluting to 0.7 mg/ml with 10 mM histidine, 135 mM sodium chloride, and pH 5.5 buffer. The cells pre-assembled in the 20°C Ti rotor were put into holes 1 and 3, and a counterweight was put into hole 4, and a cell of the same weight was put into hole 2, so that the weight difference was adjusted to be less than 0.5 g. A rotor was mounted on an analytical ultracentrifuge (Beckman coulter, OptimaAUC), and while a vacuum was maintained, scanning was performed once at 20 °C, 3,000 rpm, and 280 nm to identify whether the scan range (5.8 cm to 7.2 cm), absorbance, and analysis volume were within the normal ranges. After maintaining temperature equilibrium for 1 hour or more, the scanning was performed 150 times at 30,000 rpm at the time interval of 90 seconds at 280 nm, and when there was no change in volume during scanning, the scan result was used for Sedfit analysis. The RunID752 raw data downloaded from the analytical ultracentrifuge was identified, and the scan data from No. 1 to 130 where the experiment was conducted, was subjected to fitting by selecting a continuous c distribution model in Sedfit 16-1C, and entering a density of 0.999722 g/cm³ (Anton Paar, DMA-5000M) and a viscosity of 1.0065 The mPa·s (Anton Paar, Lovis2000ME) value as parameter values. As such, the final results were obtained.

As shown in Table 7, two AAA0589 samples with different lot numbers were tested with an analytical ultracentrifuge, and the molecular weights evaluated were 801 kDa and 776 kDa, and the multimer distribution ratios were 76% and 84%. A molecular weight evaluation value slightly smaller than the expected hexamer molecular weight of 900 kDa was obtained.

**Table 7**

| Prediction of molecular weight through analytical ultracentrifuge analysis | | |
|---|---|---|
| | AAA0589 | AAA0589 |
| Lot number | 20210127 | 20201109 |
| Sedimentation Coefficient (S) | 17.9 | 17.9 |
| Molecular Weight (kDa) | 801 | 776 |
| Ratio (%) | 75.78 | 84.02 |

### <Example 15> Dynamic light scattering analysis for confirmation of hexamer formation

Dynamic light scattering (DLS) analysis was performed to predict the molecular weight of the hexameric antibody. According to the dynamic light scattering method, the size of a particle can be predicted by measuring, over time, the wave of the intensity of light which is scattered when light hits the particle. A DynaPro's NanoStar device was used, and the sample was diluted to 0.7 mg/ml by using 10 mM histidine, 135 mM NaCl, pH 5.5 buffer, and filtered through a 0.2 µm filter, and then used for analysis. The laser having the wavelength of 662.7 nm was irradiated at 25 °C, and the measurement was performed 10 times for 5 seconds.

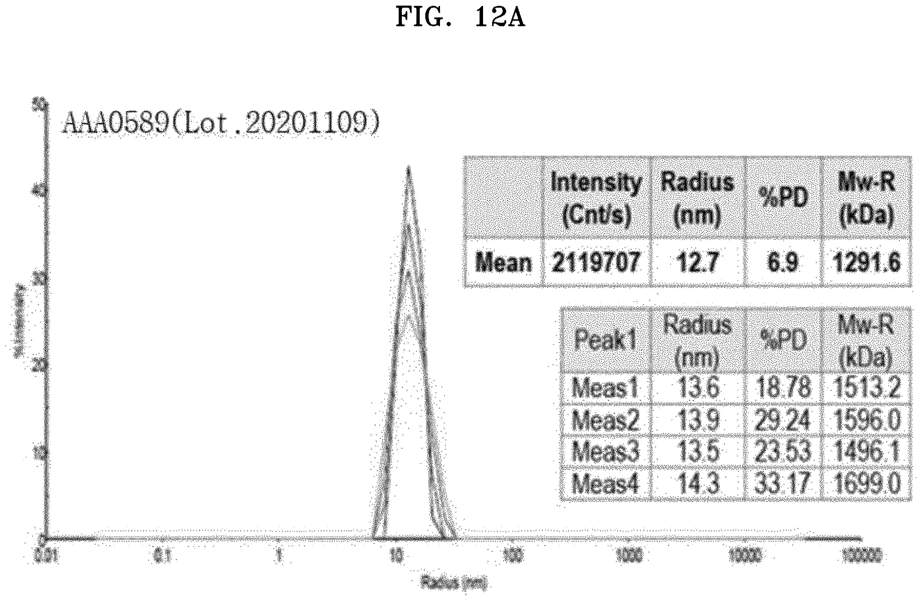

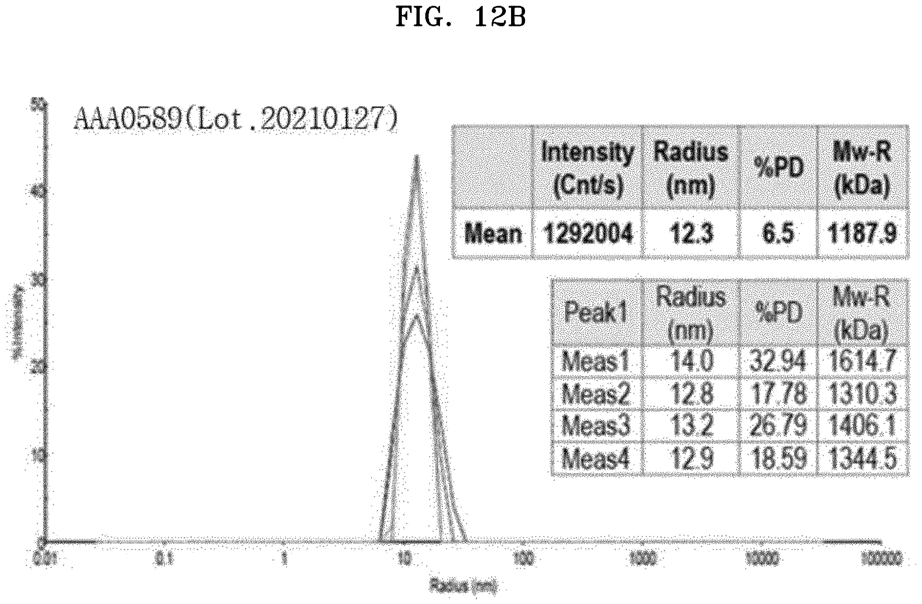

As a result of analyzing two hexamers having different Lot numbers as shown in FIGS. 12A and 12B, the molecular weight of the sample was analyzed to be 1188 kDa to 1292 kDa, and the polydispersity thereof was less than 6.9%.

## Claims

1. A hexameric antibody in which six identical antibody monomers are linked by covalent bonds, and the antibody monomers bind specifically to a vascular endothelial growth factor (VEGF).

2. The hexameric antibody of claim 1, wherein each of the antibody monomers has an isotype of immunoglobulin G (IgG).

3. The hexameric antibody of claim 2, wherein each of the antibody monomers is linked by a disulfide bond in at a CH2 region, a CH3 region, or a CH2 region and a CH3 region in an Fc region.

4. The hexameric antibody of claim 3, wherein, in each of the antibody monomers, amino acid number 309 is mutated to cysteine according to Kabat numbering; a µ tailpiece is linked to the C-terminus of Fc; or amino acid number 309 is mutated to cysteine and µ tailpiece is linked to C-terminus of Fc.

5. The hexameric antibody of claim 4, wherein, in each of the antibody monomers, according to Kabat numbering, amino acid 310 is mutated to leucine; amino acid 430 is mutated to histidine; or amino acid 310 is mutated to leucine and amino acid 430 is mutated to histidine.

6. The hexameric antibody of any one of claims 1 to 5, wherein each of the antibody monomers comprises, compared to human germ-line immunoglobulins, a variable region of a heavy chain having a heavy chain framework having one or more of Kabat numbering 13Q, 37I, 74S, 77T and 105Q; a variable region of a light chain having a light chain framework having one or more of Kabat numbering 22T, 43A, 83F and 100Q; or a combination thereof.

7. The hexameric antibody of claim 6, wherein the heavy chain framework further has one or more of 49A and 73D.

8. The hexameric antibody of any one of claims 1 to 7, wherein each of the antibody monomers comprises: a variable region of a heavy chain having one or more heavy chain frameworks of HFR1 of SEQ ID NO: 37 or SEQ ID NO: 51, HFR2 of SEQ ID NO: 38 or SEQ ID NO: 52, HFR3 of SEQ ID NO: 39 or SEQ ID NO: 53, and HFR4 of SEQ ID NO: 40; a variable region of a light chain having one or more light chain frameworks of LFR1 of SEQ ID NO: 41, LFR2 of SEQ ID NO: 42 or SEQ ID NO: 54, LFR3 of SEQ ID NO: 43, and LFR4 of SEQ ID NO: 44; or a combination thereof.

9. The hexameric antibody of claim 8, wherein each of the antibody monomers comprises a variable region of a heavy chain having a heavy chain framework consisting of HFR1 of SEQ ID NO: 37, HFR2 of SEQ ID NO: 38, HFR3 of SEQ ID NO: 39, and HFR4 of SEQ ID NO: 40; and a variable region of a light chain having a light chain framework consisting of LFR1 of SEQ ID NO: 41, LFR2 of SEQ ID NO: 42, LFR3 of SEQ ID NO: 43, and LFR4 of SEQ ID NO: 44.

10. The hexameric antibody of claim 8, wherein each of the antibody monomers comprises a variable region of a heavy chain having a heavy chain framework consisting of HFR1 of SEQ ID NO: 51, HFR2 of SEQ ID NO: 52, HFR3 of SEQ ID NO: 53, and HFR4 of SEQ ID NO: 40; and a variable region of a light chain having a light chain framework consisting of LFR1 of SEQ ID NO: 41, LFR2 of SEQ ID NO: 54, LFR3 of SEQ ID NO: 43, and LFR4 of SEQ ID NO: 44.

11. The hexameric antibody of any one of claims 1 to 10, wherein each of the antibody monomers comprises: a variable region of a heavy chain having one or more heavy chain CDR of HCDR1 of SEQ ID NO: 45 or SEQ ID NO: 55, HCDR2 of SEQ ID NO: 46 or SEQ ID NO: 56, and HCDR3 of SEQ ID NO: 47 or SEQ ID NO: 57; a variable region of a light chain having one or more light chain CDR of LCDR1 of SEQ ID NO: 48 or SEQ ID NO: 58, LCDR2 of SEQ ID NO: 49 or SEQ ID NO: 59, and LCDR3 of SEQ ID NO: 50 or SEQ ID NO: 60; or a combination thereof.

12. The hexameric antibody of claim 9 or claim 11, wherein each of the antibody monomers comprises: a variable region of a heavy chain of SEQ ID NO: 30; and a variable region of a light chain of SEQ ID NO: 31.

13. The hexameric antibody of claim 10 or claim 11, wherein each of the antibody monomers comprises: a variable region of a heavy chain of SEQ ID NO: 32; and a variable region of a light chain of SEQ ID NO: 33.

14. The hexameric antibody of claim 10 or claim 11, wherein each of the antibody monomers comprises: a variable region of a heavy chain of SEQ ID NO: 34, SEQ ID NO: 35 or SEQ ID NO: 36; and a variable region of a light chain of SEQ ID NO: 33.

15. The hexameric antibody of any one of claims 1 to 14, wherein each of the antibody monomers has a reduced or eliminated effector function.

16. The hexameric antibody of any one of claims 1 to 15, wherein each of the antibody monomers has a reduced or eliminated binding ability to one or more of FcγRI, FcγRII, FcγR III, and FcRn.

17. The hexameric antibody of any one of claims 1 to 16, wherein each of the antibody monomers has a mutation of one or more of L234A, L235A, P331G, I253A, and H310A.

18. The hexameric antibody of any one of claims 1 to 17,
wherein one of the antibody monomers has an Fc region of SEQ ID NO: 74 or SEQ ID NO: 75.

19. The hexameric antibody of claim 18, wherein each of the antibody monomers comprises a heavy chain of SEQ ID NO: 69 or SEQ ID NO: 71.

20. The hexameric antibody of claim 19, wherein each of the antibody monomers comprises a heavy chain of SEQ ID NO: 69 and a light chain of SEQ ID NO: 70.

21. The hexameric antibody of claim 19, wherein each of the antibody monomers comprises a heavy chain of SEQ ID NO: 71 and a light chain of SEQ ID NO: 72.

22. A pharmaceutical composition for preventing or treating angiogenic disease, comprising the hexameric antibody of any one of claims 1 to 21 as an active ingredient.

23. The pharmaceutical composition of claim 22, wherein the pharmaceutical composition is an aqueous pharmaceutical composition.

24. The pharmaceutical composition of claim 22 or claim 23,
wherein the angiogenic disease is an ocular disease.

25. The pharmaceutical composition of claim 24, wherein the eye disease comprises at least one selected from abnormal angiogenesis, choroid angiogenesis (CNV), retinal vascular permeability, retinal edema, diabetic retinopathy, diabetes macular edema, neovascular age-related macular degeneration (AMD), neovascular (exudative) age-related macular degeneration (AMD), sequelae associated with retinal ischemia, central retinal vein occlusion (CRVO) and posterior segment neovascularization.

26. The pharmaceutical composition of any one of claims 22 to 25, wherein the pharmaceutical composition is for a parenteral administration.

27. The pharmaceutical composition of claim 26, wherein the pharmaceutical composition is for intravitreal injection.

28. The pharmaceutical composition of any one of claims 22 to 27, being used for administration in one administration cycle every 3 months or more.

29. The pharmaceutical composition of any one of claims 22 to 28, having a concentration of at least 40 mg/ml.

30. The pharmaceutical composition of any one of claims 22 to 29, being in the form of a solution or lyophilized powder.

31. The pharmaceutical composition of any one of claims 22 to 30, being contained in a vial, an ampoule, or a pre-filled syringe.

32. An antibody composition comprising at least 90 wt% of the hexameric antibody of any one of claims 1 to 21 based on the total weight of the pharmaceutical composition.

33. The antibody composition of claim 32, comprising 95 wt% or more of the hexameric antibody.
